# EUROPEAN PATENT APPLICATION

(11) **EP 2 397 141 A1**
(43) Date of publication of application: **21.12.2011**
(21) Application number: 10166099.1
(22) Date of filing: 16.06.2010
(51) Int. Cl.: A61K 31/498, C07C 227/06, C07C 229/36

(54) **Process for the synthesis of beta-amino acids and derivatives thereof**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The present invention relates to a process for the preparation of β-amino acids and derivatives thereof, which is especially suited for gliptins and particularly sitagliptin. A key step makes use of suitably derivatized epoxides or aziridines, which owing to a chirality provides a source of chirality for intermediates which are suitable for building up β-amino acids and similar compounds, in particular in the construction of the sitagliptin molecule.

## Description

### Field of the invention

The present invention relates to the field of organic chemistry, more specifically to a process for the preparation of β-amino acids and derivatives thereof, which is especially suited for gliptins and particularly sitagliptin.

### Background of the invention

Sitagliptin is an oral antidiabetic drug, which reduces glucose blood levels by inhibition of dipeptidyl peptidase-4 (DPP-4). The mechanism of action of DPP-4 inhibitors ("gliptins") is an inactivation of glucagon-like peptide (GLP), which stimulates insulin secretion. The benefit of this medicine lays in its lower occurrence of adverse effects (e.g. hypoglycemia, weight gain) in the control of blood glucose values. Sitagliptin can be used for treatment of diabetes mellitus type 2 either alone or in combination with other oral antihyperglycemic agents, such as metformin or a thiazolidinediones.

Sitaglipin (compound of formula I) is chemically (*R*)-3-amino-1-(3-(trifluoromethyl)-5,6-dihydro-[1,2,4]triazolo[4,3-a]pyrazin-7(8H)-yl)-4-(2,4,5-trifluorophenyl)butan-1-one which structure includes a chiral β-amino acid part.

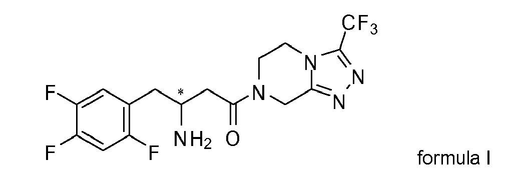

A preparation of enantiomerically pure β-amino acids and their derivatives remains a permanent challenge for industrial production. In the literature, there are several methods described, how to introduce chirality into the molecule of sitagliptin. The first synthesis of sitagliptin molecule was described in WO 03/004498 (Scheme 1) where unusual dihydropyrazine chiral promotors were used, such as diazomethane and silver salts which are unacceptable reagents for industrial synthesis.

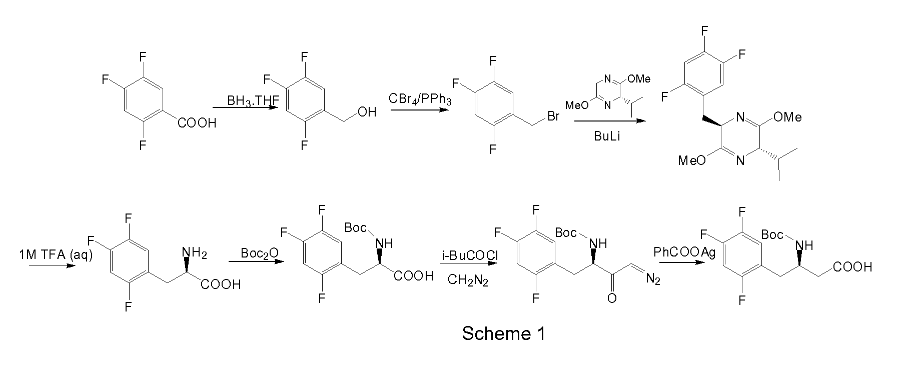

Later on, better approaches including enantioselective hydrogenation of β-enamino acid derivatives were described as presented in Scheme 2. However, these approaches require expensive precious metal catalysts, such as rhodium (WO 03/004498, Tetrahedron Asymmetry 17, 205 (2006)) or ruthenium (WO 09/064476) as well as expensive ligands, such as ferrocenyl diphosphine ligands - JOSIPHOS catalysts (WO 04/085378, WO 05/097733, WO 06/081151, J. Am. Chem. Soc., 126, 9918 (2004*)*).

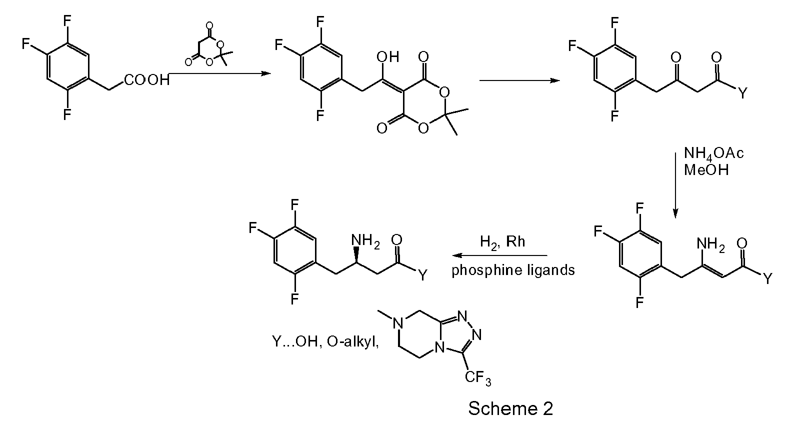

Another option described in WO 04/085661 is a hydrogenation process using a less expensive achiral catalyst as presented in Scheme 3. However, chiral derivatisation of enamines derived from phenylglycinamide obtained enantiomeric excess (e. e.) values that are not sufficient for pharmaceutical use.

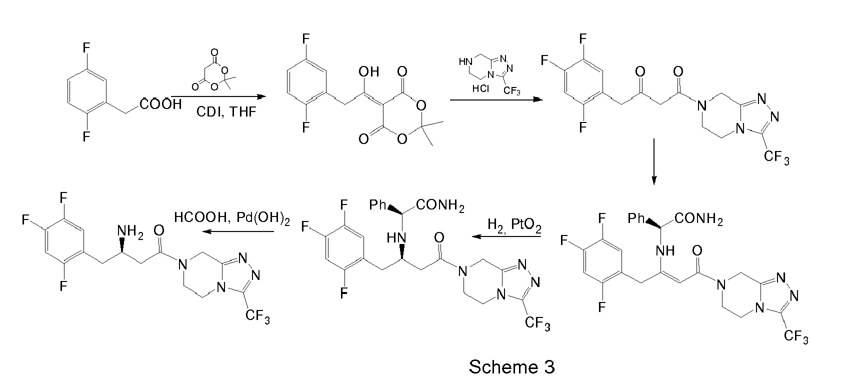

Yet another option is introducing chiral centers by selective reduction of β-keto acid derivatives using precious metal catalysts (WO 04/087650, Org. Prep. Res. & Dev. 9, 634 (2005)) or enzymatic reduction (WO 09/045507). However, the transformation of the obtained chiral hydroxyl intermediates to final sitagliptin precursors via azetidinone intermediates is laborious as presented in Scheme 4.

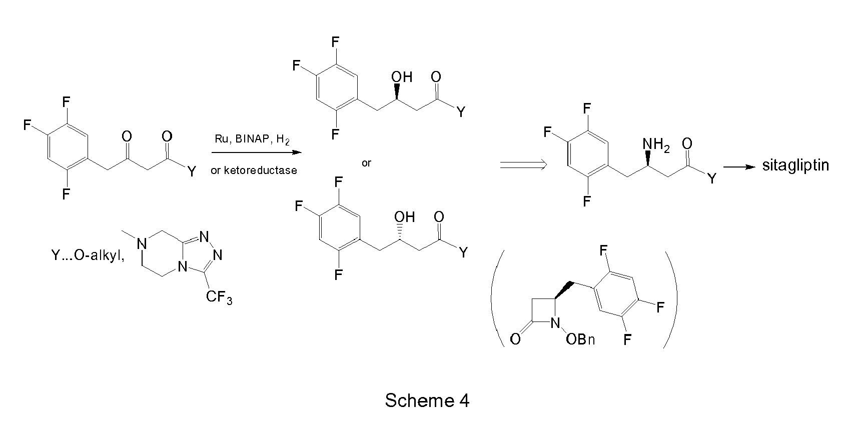

There is a continued need, and therefore there it is an object of the present invention to provide an efficient route for the synthesis of β-amino acid derivatives, in particular for the synthesis of gliptins and especially of sitagliptin.

### Summary of the invention

The present invention provides the following items including main aspects and preferred embodiments, which respectively alone and in combination particularly contribute to solving the above object and eventually provide additional advantages:
1. A process of preparing a compound of formula IV, 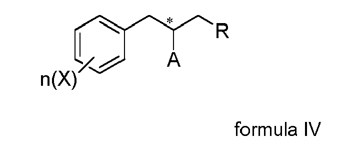 wherein X is halogen selected from fluoro, chloro, or bromo, preferably fluoro, same or different, and n is 1-4, A is OH or NH2_{,} * denotes a chiral atom and R is selected from
   a leaving group or a group convertible to a leaving group;
   the process comprising the steps of:
   a) providing a compound of formula II, 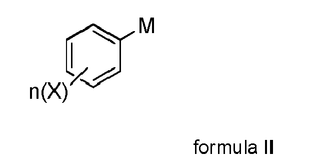 wherein X and n are as defined above and M is a metal element or an organometal group;
   b) reacting of the compound of formula II with a compound of formula IIIa when A is OH or with a compound of formula IIIb when A is NHP, wherein P is an amine protecting group and/or after deprotection NH₂, 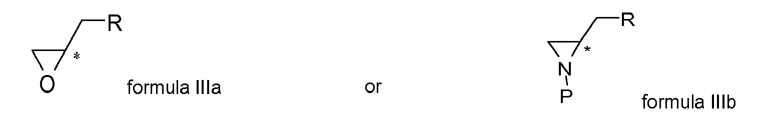 wherein * denotes a chiral atom and R is as defined above.
2. The process according to item 1, wherein A is OH and the compound of formula II is reacted with the compound of formula IIIa.
3. The process according to any of the preceding items, wherein the compound of formula II is defined by formula IIa, 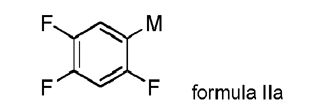 wherein M is as defined above, and wherein the compound of formula IIa is provided by treating a halo-trifluorobenzene, wherein "halo" means chloro, bromo or iodo, preferably chloro, with a metal or an organometal compound of M to form a compound of formula II with X being F and n being 3.
4. The process according to item 3, wherein the reaction to obtain the compound of formula IIa and the reaction of the compound of formula IIa with the compound of formula IIIa proceed in one pot.
5. The process according to any of the preceding items, wherein R is a leaving group selected from the group consisting of halogen, arylsulfonyloxy, unsubstituted and fluorinated C₁-C₄-alkylsulfonyloxy.
6. The process according to any of the preceding items, wherein the leaving group R is selected from halogen, methanesulfonyl and p-substituted benzenesulfonyl group, preferably chloro.
7. The process according to any of the preceding items, wherein M is or contains magnesium, zinc or lithium, preferably M is halomagnesio.
8. The process according to any of the preceding items, wherein reaction step b) is carried out in the presence of catalytic amounts of copper, manganese or cerium salts, preferably copper salts.
9. The process according to any of the preceding items, wherein R is a group convertible to a leaving group, selected from protected hydroxy groups.
10. The process according to any of the preceding items, wherein R is a group which is inert against organometals and which is convertible to a group selected from cyano, aminocarbonyl, hydroxycarbonyl and alkoxycarbonyl groups.
11. The process according to item 10, wherein R is 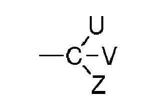 wherein U, V, and Z comprises
   0-2 hydrogen atoms,
   1-3 substituents selected from 1-oxa, 1-aza, 1-thia C₁-C₆-alkyl substituents in which two of them may be coupled to a 5-7-membered ring, or two of them may be joined to one and bound to carbon atom through a double bond, C₁-C₂-alkylidene.
12. The process according to item 11, wherein U, V, Z are trialkoxy characterizing orthoesters, U, V, Z are dialkoxy or alkylenedioxy characterizing acetals, U, V, Z are dialkylthio or alkylenedithio characterizing thioacetals, or U, V, Z are bis(dialkylamino or alkylenediamino characterizing aminals.
13. The process according to any of the proceeding items, wherein the compound of formula IIIa is in the (S)-configuration, preferably (*S*)-epichlorohydrin is used as the compound of formula IIIa to obtain 1-chloro-3-(2,4,5-trifluorophenyl)-2(*S*)-propanol as the compound of formula IV.
14. The process according to any one of items 1 to 8, wherein the compound of formula IV with A being OH, 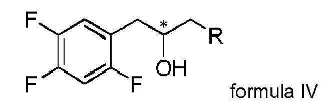 wherein * denotes a chiral atom and R is a leaving group selected from halogen, arylsulfonyloxy, unsubstituted or fluorinated C₁-C₄-alkylsulfonyloxy, preferably chloro,
   is further converted to a compound of formula V, 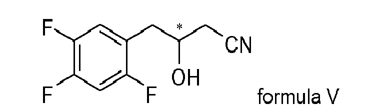 wherein * denotes a chiral atom
   by treatment with cyanides, selected from alkali, earth-alkali metalic or quaternary ammonium cyanides.
15. The process according to item 14, wherein the further conversion to the compound of formula V is carried out by treatment with potassium or sodium cyanide in a protic solvent or in a mixture of protic and aprotic solvent, preferably in a water/N,N-dimethylformamide mixture.
16. The process according to any one of items 1 to 10, wherein the compound of formula IV with A being OH, 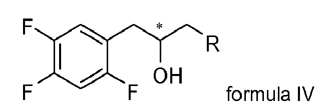 wherein * denotes a chiral atom and R is a hydroxy group, optionally protected to be inert in an organometal containing medium, is further converted to a compound of formula V, 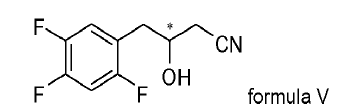 wherein * denotes a chiral atom and said process comprises the steps of:
   a) optionally deprotecting the protected hydroxy group,
   b) transforming the hydroxyl group to halogen, arylsulfonyloxy, unsubstituted or fluorinated C₁-C₄-alkylsulfonyloxy group, and
   c) treating the obtained compound of formula IV with cyanide anion selected from alkali, earth-alkali metalic or quaternary ammonium cyanides, preferably by treatment with potassium or sodium cyanide in a protic solvent or in a mixture of protic and aprotic solvent.
17. The process according to any one of items 14 to 16, wherein the compound of formula V is further converted to a compound of formula VI, 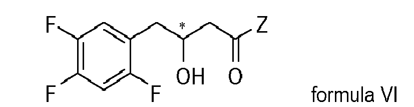 wherein * denotes a chiral atom and Z is selected from the group consisting of hydroxy, amino, OR₁, wherein R₁ is selected from C₁-C₆-alkyl and halo substituted C₁-C₆-alkyl, and NH₂
   by treatment with strong acid.
18. The process according to item 17, wherein the strong acid is selected from the group consisting of hydrogen halogenide, preferably HCl, perchloric, alkanesulfonic, halo substituted alkanesulfonic, arylsulfonic acids and sulfonic acids on polymer support.
19. The process according to item 11 or 12, wherein R is a group converted to C(=O)Z depicted in the compound of formula VI, and Z is selected from the group consisting of hydroxy, amino, OR₁, wherein R₁ is selected from C₁-C₆-alkyl and halo substituted C₁-C₆-alkyl, and NHR₂, wherein R₂ is selected from H, C₁-C₄-alkyl, aryl-C₁-C₂-alkyl, C₁-C₄-alkoxy, aryl-C₁-C₂-alkoxy by reactions of hydrolysis, alcoholysis, oxidation, or ozonization.
20. The process according to any one of items 14 to 18, wherein the compound of formula V or a salt thereof is further converted to a compound of formula Vlc, 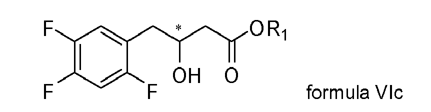 wherein * denotes a chiral atom and R₁ is selected from the group consisting of C₁-C₆-alkyl or halo substituted C₁-C₆-alkyl,
   by treatment with a strong acid, preferably a strong acid as defined in item 18, in a solvent of formula R₁OH, wherein R₁ is H, C₁-C₆-alkyl or halo substituted C₁-C₆-alkyl, preferably ethanol or methanol, optionally diluted with water or aprotic organic solvent.
21. The process according to any one of items 14 to 17, wherein the compound of formula V is further converted to a compound of formula VIa 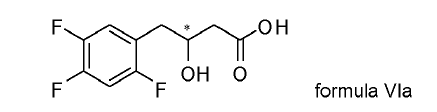 wherein * denotes a chiral atom
   by a treatment selected from a) to c):
   a) treating the compound of formula V in aqueous alkaline conditions, optionally diluted with water miscible solvent, preferably in the presence of hydrogen peroxide, more preferably in concentrated ammonia in the presence of hydrogen peroxide,
   b) treating the compound of formula V with a strong acid, preferably a strong acid as defined in item 18,
   c) treating the compound of formula VIc, 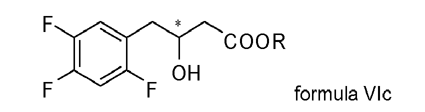 wherein * denotes a chiral atom and R is C₁-C₆-alkyl or halo substituted C₁-C₆-alkyl, with an aqueous base selected from the group of alkali hydroxides, earth-metal hydroxides and organic bases, preferably sodium hydroxide in C₁-C₄-alkanol/water mixture, most preferably sodium hydroxide in a methanol/water mixture.
      Here, preference of treatments is increased from a) to c).
22. The process according to item 21, wherein the compound of formula VIa is further converted to a compound of formula VId, 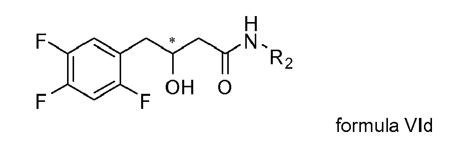 wherein * denotes a chiral atom and R₂ is selected from H, C₁-C₄-alkyl, aryl-C₁-C₂-alkyl, C₁-C₄-alkoxy, aryl-C₁-C₂-alkoxy, trifluoroacetyl,
   by treatment with a coupling reagent selected from carbodiimides and amines R₂-NH₂, wherein R₂ is selected from H, C₁-C₄-alkyl, aryl-C₁-C₂-alkyl, C₁-C₄-alkoxy, aryl-C₁-C₂-alkoxy and trifluoroacetyl, in the presence of a solvent.
23. The process according to item 22, wherein the coupling reagent is selected from N,N'-dicyclohexylcarbodiimide, N,N'-diisopropylcarbodiimide, N-ethyl-N'-(3-dimethyamino propyl)carbodiimide and 1-hydroxybenzotriazole, and/or the solvent is selected from haloalkanes, nitriles, alkylcarboxylic esters, ethers, aromatic hydrocarbons and mixtures thereof.
24. The process according to item 21, wherein the treatment according to a) or b) is performed to obtain a compound of formula VIb 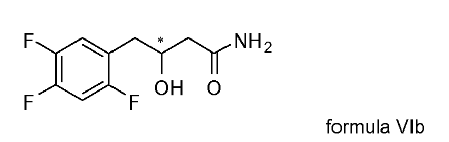 wherein * denotes a chiral atom.
25. The process according to any of items 17, 19 or 21, further comprising the steps of:
   a) reacting the compound of formula VI, wherein Z is hydroxyl, or the compound of formula V, with a heterocyclic moiety of formula VIII 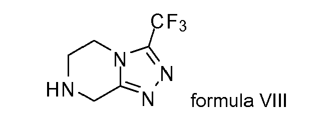 in the presence of coupling reagent in an organic solvent, preferably using a coupling reagent in an organic solvent as defined in item 23, to form a compound of formula VII 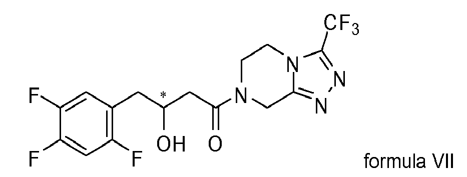 wherein * denotes a chiral atom
   b) sulfonation of the compound of formula VII or a salt thereof with a halogenide or an anhydride of a sulfonic acid in the presence of a base, preferably a weak base, in an aprotic organic solvent,
      to obtain a compound of formula IX or a salt thereof 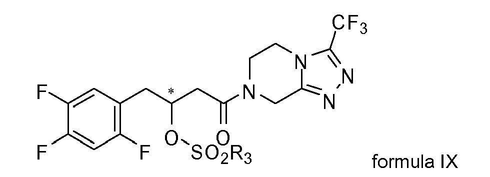 wherein * denotes a chiral atom and R₃ is selected from methyl, ethyl, benzyl, p-methylbenzyl, p-bromobenyl, p-nitrobenzyl and 10-camphoryl.
26. The process according to item 25 further comprising
   a) treatment of the compound of formula IX with an azide reagent, preferably alkali metal azide or trialkylammonium azide to obtain a compound of formula X, 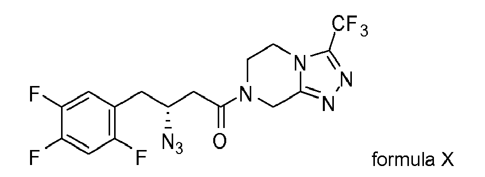 wherein chiral atom denoted by * has the opposite configuration than the analogue of compound of IX
   b) further reducing azido group to obtain compound of formula I, 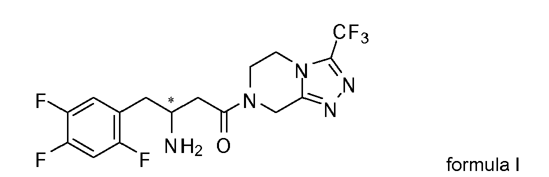 wherein chiral atom denoted by * has the chiral configuration of the compound of formula X.
27. The process according to any one of items 17 to 19 or 21, further comprising sulfonation of the compound of formula VI with a halogenide or an anhydride of sulfonic acids, in the presence of a base, preferably a weak base, in an aprotic organic solvent, to obtain a compound of formula XI, 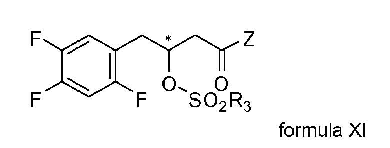 wherein * denotes a chiral atom and Z is selected from the group consisting of OR₁, wherein R₁ is selected from C₁-C₆-alkyl and halo substituted C₁-C₆-alkyl, and NHR₂, wherein R₂ is selected from H, C₁-C₄-alkyl, aryl-C₁-C₂-alkyl, C₁-C₄-alkoxy, aryl-C₁-C₂-alkoxyand trifluoroacetyl, and R₃ is selected from methyl, ethyl, benzyl, p-methylbenzyl, p-bromobenyl, p-nitrobenzyl and 10-camphoryl.
28. The process according to item 27, wherein the compound of formula XI, 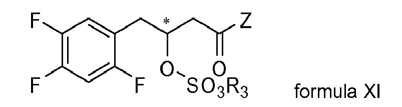 wherein Z is selected from the group consisting of OR₁, wherein R₁ is selected from C₁-C₆-alkyl and halo substituted C₁-C₆-alkyl,
   comprising the steps consisting of
   a) treatment with alkali metal azide or trialkylammonium azide to obtain a compound of formula XII, 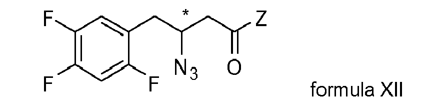 wherein chiral atom denoted by * has the opposite configuration of a compound of XI and Z is same as above;
   b) reacting of the compound of formula XII with a heterocyclic moiety of formula VIII, 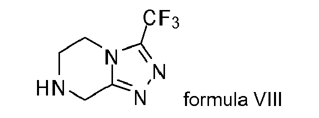 in the presence of coupling reagent in an organic solvent, preferably using a coupling reagent in an organic solvent according to item 23, to obtain a compound of formula X, 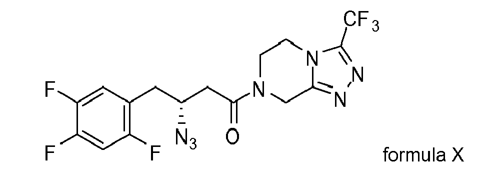
   c) further reducing azido group to obtain the compound of formula 1, 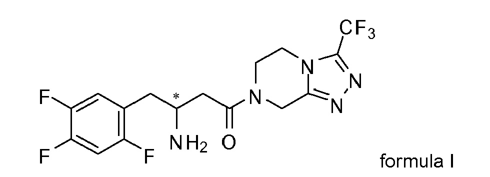 wherein chiral atom denoted by * has a chiral configuration of the compound of formula XII.
29. The process according to any of the items 25 to 28, wherein the halogenide or the anhydride of sulfonic acids is a halogenide or an anhydride of methanesulfonic, ethanesulfonic, benzenesulfonic, p-toluenesulfonic, p-bromobenzenesulfonic, p-nitrobenzenesulfonic or camphor-10-sulfonic, and wherein the aprotic organic solvent is selected from carboxylic esters, haloalkanes, aromatic hydrocarbons, alkyl ethers, nitriles and N,N-dialkylamides.
30. A compound of formula IV, 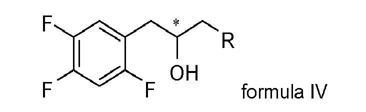 wherein * denotes a chiral atom and R is selected from
   a leaving group, or
   a group convertible to a leaving group.
31. The compound according to item 30, wherein R is a group selected from the group consisting of halogen, arylsulfonyloxy, unsubstituted and fluorinated C₁-C₄-alkylsulfonyloxy.
32. The compound according to item 30, wherein R is a hydroxy group, optionally protected to be inert in an organometal containing medium, or an organometal inert group convertible to a group selected from cyano aminocarbonyl, hydroxycarbonyl and alkoxycarbonyl.
33. A compound of formula VI, 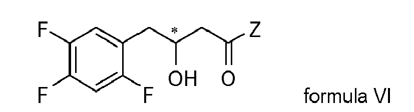 wherein * denotes a chiral atom and Z is selected from the group consisting of NHR₂, wherein R₂ is selected from H, C₁-C₄-alkyl, aryl-C₁-C₂-alkyl, C₁-C₄-alkoxy and trifluoroacetyl.
34. A compound of formula XI, 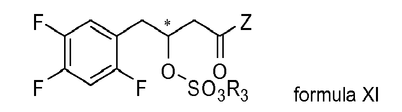 wherein * denotes a chiral atom and Z is selected from the group consisting of OR₁, wherein R₁ is selected from C₁-C₆-alkyl or halo substituted C₁-C₆-alkyl, NHR₂, wherein R₂ is selected from H, C₁-C₄-alkyl, aryl-C₁-C₂-alkyl, C₁-C₄-alkoxy, aryl-C₁-C₂-alkoxy, trifluoroacetyl and R₃ is selected from methyl, ethyl, benzyl, p-methylbenzyl, p-bromobenzyl, p-nitrobenzyl and 10-camphoryl.
35. The process according to item 27, wherein the compound of formula XI, 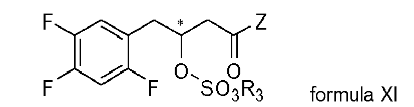 wherein Z is selected from the group consisting of NHR₂, and R₂ is selected from C₁-C₆-alkyl, aryl-C₁-C₄-alkyl, C₁-C₄-alkoxy and trifluoroacetyl is further converted to a compound of formula XIV, 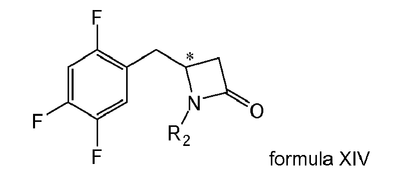 wherein chiral atom denoted by * has the opposite configuration of the analogue of compound of XI and R₂ is selected from C₁-C₆-alkyl, aryl-C₁-C₄-alkyl and trifluoroacetyl
   by cyclization of in the presence of strong base, preferably sodium hydride, in a solvent selected from amides, halogenated alkanes and aromatic hydrocarbons.
36. A compound of formula XIV, 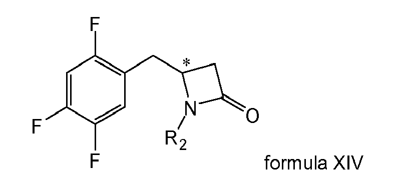 wherein R₂ is selected from C₁-C₆-alkyl, aryl-C₁-C₄-alkyl, C₁-C₄-alkoxy and trifluoroacetyl.
37. A compound of formula XVII, 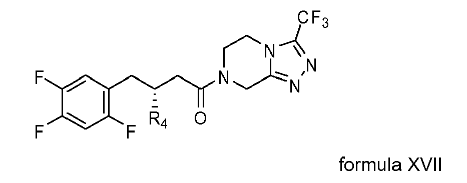 wherein R₄ is selected from azido and NHOMe.
38. A process for producing sitagliptin or a salt thereof, comprising the steps of carrying out a process for preparing the compound of formula IV according to item 1, or carrying out a process for preparing the compound of formula VI according to item 17 or 19,
   and subjecting the compound of formula IV or the compound of formula VI to further synthesis steps to yield sitagliptin or a salt thereof.
39. The process according to item 38, comprising the steps of:
   a) using a compound of formula IIIa, wherein * denotes a chiral atom of (*S*) configuration,
   b) providing a compound of formula IV prepared by a process according to any one of items 1 to 13,
   c) converting said compound of formula IV to a compound of formula VI by a process according to any one of items 17 to 19,
   d) converting said compound of formula VI to a compound of formula IX by a process according to item 25,
   e) converting said compound of formula IX to sitagliptin or salt thereof.
40. The process according to item 38, comprising the steps of:
   a) using a compound of formula IIIa, wherein * denotes a chiral atom of *(S)* configuration,
   b) providing a compound of formula IV prepared by a process according to any one of items 1 to 13,
   c) converting said compound of formula IV to a compound of formula VI by a process according to any one of items 17 to 19,
   d) converting said compound of formula VI to a compound of formula IX by a process according to item 25,
   e) converting said compound of formula IX to a compound of formula X by a process according to item 26,
   f) converting said compound of formula X to sitagliptin or salt thereof.
41. The process according to item 38, comprising the steps of:
   a) using a compound of formula Ilia, wherein * denotes a chiral atom of *(S)* configuration,
   b) providing a compound of formula IV prepared by a process according to any one of items 1 to 13,
   c) converting said compound of formula IV to a compound of formula VI by a process according to any one of items 17 to 19,
   d) converting said compound of formula VI to a compound of formula XI by a process according to item 27,
   e) converting said compound of formula XI to a compound of formula XIV by a process according to item 35,
   f) converting said compound of formula XIV to sitagliptin or salt thereof.
42. Use of a compound of formula IV, 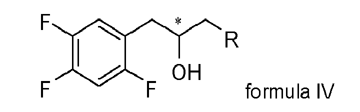 wherein * denotes a chiral atom and R is selected from leaving groups or groups convertible to a leaving group for the synthesis of gliptins or salts thereof.
43. Use of a compound of formula VI, 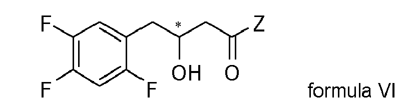 wherein * denotes a chiral atom and Z is selected from the group consisting of OR₁, wherein R₁ is selected from C₁-C₆-alkyl and halo substituted C₁-C₆-alkyl, NHR₂, wherein R₂ is selected from H, C₁-C₄-alkyl, aryl-C₁-C₂-alkyl, C₁-C₄-alkoxy, aryl-C₁-C₂-alkoxy and trifluoroacetyl,
   for the synthesis of gliptins or salts thereof.
44. Use of a compound of formula XI, 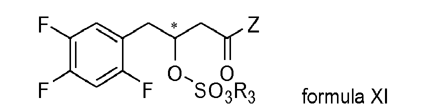 wherein * denotes a chiral atom and Z is selected from the group consisting of OR₁, wherein R₁ is selected from C₁-C₆-alkyl or halo substituted C₁-C₆-alkyl, NHR₂, wherein R₂ is selected from H, C₁-C₄-alkyl, aryl-C₁-C₂-alkyl, C₁-C₄-alkoxy, aryl-C₁-C₂-alkoxy, trifluoroacetyl and R₃ is selected from methyl, ethyl, benzyl, p-methylbenzyl, p-bromobenzyl, p-nitrobenzyl and 10-camphoryl
   for the synthesis of gliptins or salts thereof.
45. Use of a compound of formula XIV, 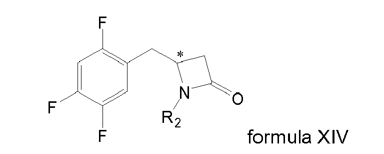 wherein R₂ is selected from C₁-C₆-alkyl, aryl-C₁-C₄-alkyl, C₁-C₄-alkoxy and trifluoroacetyl,, for the synthesis of gliptins or salts thereof.
46. Use of a compound of formula XVII, 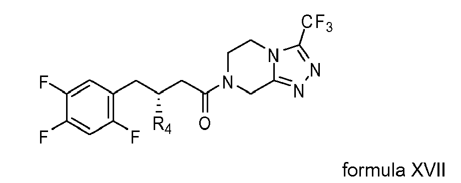 wherein R₄ is selected from azido and NHOMe.
   for the synthesis of gliptins or salts thereof.
47. A process for producing a pharmaceutical composition comprising sitagliptin as active ingredient, comprising the steps of: a) preparing sitagliptin or a salt thereof according to a process as defined in any one of items 38 to 41, and b) admixing the thus prepared sitagliptin with at least one pharmaceutically acceptable excipient.

### Detailed description of the invention

In the following, the present invention will be described in more details by preferred embodiments and examples while referring to the attached drawings, noting however, that these embodiments, examples and drawings are presented for illustrative purposes only and shall not limit the invention in any way.

The present invention provides an industrially applicable, economical and acceptable process for eventually yielding β-amino acids, particularly suitable for obtaining gliptins and especially for synthesizing sitagliptin or salts thereof such as the phosphate salt. The process according to the present invention makes use of inexpensive commercial chiral starting materials for introducing chirality into the desired β-amino acid molecule such as sitagliptin and its variants and derivatives. Advantageously the present invention allows an efficient, yet highly selective inclusion of the chiral center already at an early stage in the synthesis of β-amino acid derivatives, using chiral epoxides or chiral aziridines as a source of chirality for intermediates which are suitable for building up β-amino acids and similar compounds, in particular in the construction of the sitagliptin molecule. In a preferred and advantageous embodiment particularly suitable to prepare sitagliptin, the present invention makes use of chiral epoxides, in particular epichlorohydrine, glycidol, etc., as respective easily accessible, economically and synthetically advantageous raw materials widely used in the chemical and pharmaceutical industry.

The term "alkyl" as used herein, if not stated otherwise with respect to particular embodiments, include reference to a straight or branched chain alkyl moiety having 1, 2, 3, 4, 5 or 6 carbon atoms. This term includes methyl, ethyl, propyl (n-propyl or isopropyl), butyl (n-butyl, sec-butyl or tert-butyl), pentyl, hexyl and the like. In particular, alkyl may have 1, 2, 3 or 4 carbon atoms.

The term "cycloalkyl" as used herein, if not stated otherwise with respect to particular embodiments, includes an alicyclic moiety having 3, 4, 5, 6, 7 or 8 carbon atoms. The group may be a bridged or polycyclic ring system. More often cycloalkyl groups are monocyclic. This term includes groups such as cyclopropyl, cyclobutyl, cyclopentyl, cyclohexyl, norbomyl, bicyclo[2.2.2]octyl and the like.

The term "alkoxy" as used herein, if not stated otherwise with respect to particular embodiments, include -O-alkyl, wherein alkyl is straight or branched chain and comprises 1, 2, 3, 4, 5 or 6 carbon atoms. In certain embodiments, alkoxy has 1, 2, 3 or 4 carbon atoms. This term includes methoxy, ethoxy, propoxy, isopropoxy, butoxy, tert-butoxy, pentoxy, hexoxy and the like.

The term "aryl" as used herein, if not stated otherwise with respect to particular embodiments, includes reference to an aromatic ring system comprising 6, 7, 8, 9, 10, 11, 12, 13, 14, 15 or 16 ring carbon atoms. Aryl is often phenyl but may be a polycyclic ring system, having two or more rings, at least one of which is aromatic. This term includes phenyl, naphthyl, fluorenyl, azulenyl, indenyl, anthryl and the like.

The term "heterocycle" as used herein includes, if not stated otherwise with respect to particular embodiments, a saturated (e.g. heterocycloalkyl) or unsaturated (e.g. heteroaryl) heterocyclic ring moiety having 3, 4, 5, 6, 7, 8, 9, 10, 11, 12, 13, 14, 15 or 16 ring atoms, at least one of which is selected from nitrogen and oxygen. In particular, heterocyclyl includes a 3- to 10-membered ring or ring system and more particularly a 5- or 6- or 7-membered ring, which may be saturated or unsaturated; examples thereof include oxiranyl, azirinyl, 1,2-oxathiolanyl, imidazolyl, thienyl, furyl, tetrahydrofuryl, pyranyl, thiopyranyl, thianthrenyl, isobenzofuranyl, benzofuranyl, chromenyl, 2H-pyrrolyl, pyrrolyl, pyrrolinyl, pyrrolidinyl, imidazolyl, imidazolidinyl, benzimidazolyl, pyrazolyl, pyrazinyl, pyrazolidinyl, thiazolyl, isothiazolyl, dithiazolyl, oxazolyl, isoxazolyl, pyridyl, pyrazinyl, pyrimidinyl, piperidyl, piperazinyl, pyridazinyl, morpholinyl, thiomorpholinyl, especially thiomorpholino, indolizinyl, isoindolyl, 3H-indolyl, indolyl, benzimidazolyl, cumaryl, indazolyl, triazolyl, tetrazolyl, purinyl, 4H-quinolizinyl, isoquinolyl, quinolyl, tetrahydroquinolyl, tetrahydroisoquinolyl, decahydroquinolyl, octahydroisoquinolyl, benzofuranyl, dibenzofuranyl, benzothiophenyl, dibenzothiophenyl, phthalazinyl, naphthyridinyl, quinoxalyl, quinazolinyl, quinazolinyl, cinnolinyl, pteridinyl, carbazolyl, ß-carbolinyl, phenanthridinyl, acridinyl, perimidinyl, phenanthrolinyl, furazanyl, phenazinyl, phenothiazinyl, phenoxazinyl, ehromenyl, isochromanyl, chromanyl and the like.

More specifically, a saturated heterocyclic moiety may have 3, 4, 5, 6 or 7 ring carbon atoms and 1, 2, 3, 4 or 5 ring heteroatoms selected from nitrogen and oxygen. The group may be a polycyclic ring system but more often is monocyclic, for example including azetidinyl, pyrrolidinyl, tetrahydrofuranyl, piperidinyl, oxiranyl, pyrazolidinyl, imidazolyl, indolizidinyl, piperazinyl, thiazolidinyl, morpholinyl, thiomorpholinyl, quinolinidinyl and the like. Furthermore, the "heteroaryl" may include an aromatic heterocyclic ring system having 5, 6, 7, 8, 9, 10, 11, 12, 13, 14, 15 or 16 ring atoms, at least one of which is selected from nitrogen and oxygen. The group may be a polycyclic ring system, having two or more rings, at least one of which is aromatic, but is more often monocyclic. This term includes pyrimidinyl, furanyl, benzo[b]thiophenyl, thiophenyl, pyrrolyl, imidazolyl, pyrrolidinyl, pyridinyl, benzo[b]furanyl, pyrazinyl, purinyl, indolyl, benzimidazolyl, quinolinyl, phenothiazinyl, triazinyl, phthalazinyl, 2Hehromenyl, oxazolyl, isoxazolyl, thiazolyl, isoindolyl, indazolyl, purinyl, isoquinolinyl, quinazolinyl, pteridinyl and the like.

The term "substituted" as used herein in reference to a structure/moiety/group means that one or more, especially up to 5, more especially 1, 2 or 3, of the hydrogen atoms in said structure/moiety/group are replaced independently of each other by the corresponding number of substituents known to a person skilled in the art. Typical substituents include, without being limited to halogen, trifluoromethyl, cyano, nitro, oxo, NR', -OR', -C(O)R', -C(O)OR', -OC(O)R', -S(O)R', N(R')R", C(O)N(R')R", -SO₂N(R')R' and R"', wherein each of R', R" and R'" are selected from the group consisting of C1 - C6 alkyl, C1 - C6 alkoxy, -(CH₂)m-heterocyclyl (m being 1, 2, 4 or 4) and each R' and R" may be optionally and independently further substituted with one or more of hydrogen, halogen, cyano, amino, hydroxy, C1 - C6 alkyl and C1 - C6 alkoxy. Specific substituents in particular include halogen such as fluoro, chloro and/or bromo, hydroxy, amino, C1 - C6 alkyl and C1 - C6 alkoxy, and halogenated C1 - C6 alkyl and C1 - C6 alkoxy such as trifluoro-methyl. It will be understood that substituents are at positions where they are chemically possible, it being known or evident to the person skilled in the art to decide (either experimentally or theoretically) without inappropriate effort whether a particular substitution is possible. For example, substituents which may be unstable or may affect reactions disclosed herein may be omitted, at least at the relevant stage of intermediate compound or of the affected reaction.

The term "base substance" used herein according to preferred embodiments can be any base known and typically used in organic synthesis. The base can include, without being limited to, amides, hydrides, hydroxides, amidines, tertiary amines. The preferred base is selected from hydroxides, ammonia, amines, and inorganic basic salts such as acetates, carbonates, phosphates.

The terms "leaving group" and "a group convertible to a leaving group" used herein have their typical meaning known to those skilled in organic synthesis. A "group convertible to a leaving group" has the capability of being conventionally converted to a desired leaving group. Preferably and in view of the key reaction step of the present invention, the leaving group and the group convertible to a leaving group respectively are inert in an metal- or organometal-containing medium. Optionally the respective leaving group of a precursor group thereof is protected by usual and known protection groups. The respective meanings of these terms become further apparent from the more specific definitions provided herein in the disclosure of preferred embodiments.

According to a preferred option (A) of an embodiment of the present invention for the synthesis of a DPP-4 inhibitor of formula 1, 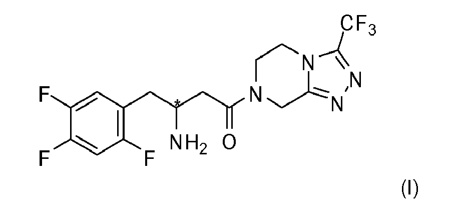 preferably sitagliptin as its *(S)* enantiomer
comprises
a) preparing a compound of formula IIa, 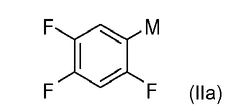 wherein M is an metalo or organometal residue, preferably M is MgX, ZnX or Li and X is Cl, Br, I
   by treatment of a halo-trifluorobenzene with an appropriate metal or organometal compound, for example selected from the group consisting of alkyl metals, aryl metals and halides thereof, such as alkyl magnesium chloride, alkyl zinc chloride and lithium chloride, wherein alkyl suitably is C₁-C₆;
b) reacting of the compound of formula IIa with a compound of formula IIIa, 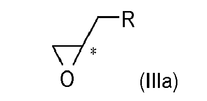 wherein * denotes a chiral atom and R is Cl or Br
   to form halohydrine of formula IV, 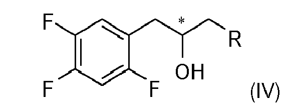 wherein * denotes a chiral atom and R is Cl or Br, optionally in the presence of catalytic amounts of copper (I) salts, preferably Cu₂X'₂, wherein X' is Cl, Br or I;
c) converting halohydrin of formula IV to cyanohydrin of formula V 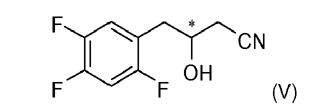 wherein * denotes a chiral atom
   by reacting with cyanide ion in a protic solvent or in a mixture of protic and aprotic solvent, preferably in a water/N,N-dimethylformamide mixture;
d) hydrolyzing cyanohydrin of formula V to an ester of formula VIc, 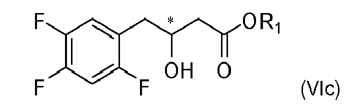 wherein * denotes a chiral atom and R₁ is C₁-C₆-alkyl or halo substituted C₁-C₆-alkyl,
   by treatment with a strong acid in a C₁-C₆-alkanol or halo substituted C₁-C₆-alkanol;
e) saponification of the ester of formula VIc to an acid of formula VIa, 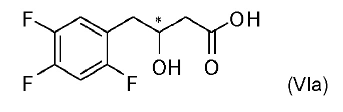 wherein * denotes a chiral atom
   by hydroxide solution in C₁-C₄-alkanol
f) reacting the acid of formula VIa with a compound of formula VIII 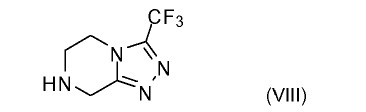 in the presence of coupling reagent in a suitable organic solvent
   producing a compound of formula VII, 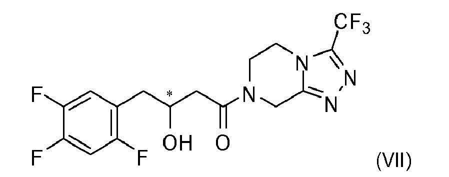 wherein * denotes a chiral atom.
g) sulfonation of compound of formula VII to a compound of formula IX 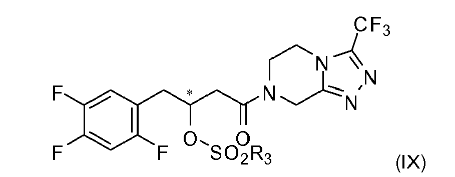 wherein * denotes a chiral atom and R₃ is selected from methyl, ethyl, benzyl, p-methylbenzyl, p-bromobenzyl, p-nitrobenzyl and 10-camphoryl;
   by treatment with halogenides or anhydrides of a sulfonic acid in the presence of weak bases in an aprotic organic solvent;
h) converting the compound of formula IX to a compound of formula X 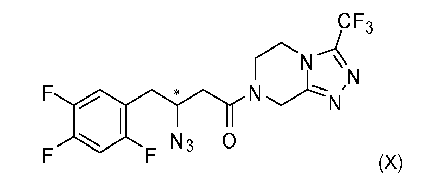 wherein * denotes a chiral atom
   by treatment with an azide reagent with complete or predominate conversion of chirality;
i) reducing the compound of formula X into the compound of formula I 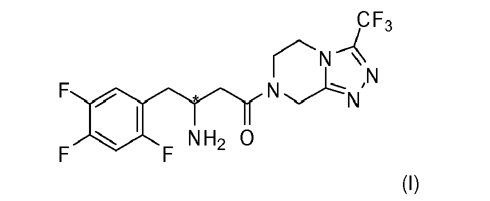 wherein * denotes a chiral atom
   by catalytic hydrogenation or reaction with trisubstituted phosphines.

According to another option (B) of the embodiment present invention for the synthesis of sitagliptin, the compound of formula VIc of the step d) is isolated.

According to another option (C) of the embodiment present invention for the synthesis of sitagliptin, the compound of formula VIc of the step d) is not isolated but after neutralization of the reaction mixture and optional extraction solvents are partially or completely removed and the residue is transferred to conditions of the reaction of step e).

According to an alternative option (D) of the embodiment present invention for the synthesis of sitagliptin, the reaction step d) is carried out in the absence of alcohol. In such aspect of the invention the compound of formula VIa could be prepared without providing step e) or optionally by applying step e) in order to reduce an amount of partial hydrolytic products such as amide of formula VIb.

In another optional embodiment (E) steps e) to h) of the option (A) are replaced by
j) sulfonation the compound of formula VIc, 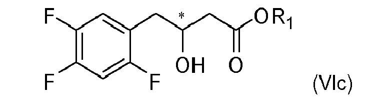 wherein * denotes a chiral atom and R₁ is C₁-C₆-alkyl or halo substituted C₁-C₆-alkyl,
   by treatment with halogenides or anhydrides of a sulfonic acid in the presence of a weak base in an aprotic organic solvent
   to form a compound of formula XI, 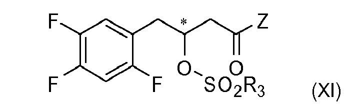 wherein * denotes a chiral atom and Z is selected from the group consisting of OR₁, wherein R₁ is selected from C₁-C₆-alkyl or halo substituted C₁-C₆-alkyl, NHR₂, wherein R₂ is selected from H, C₁-C₄-alkyl, aryl-C₁-C₂-alkyl, C₁-C₄-alkoxy, aryl-C₁-C₂-alkoxy, trifluoroacetyl and R₃ is selected from methyl, ethyl, benzyl, p-methylbenzyl, p-bromobenyl, p-nitrobenzyl and 10-camphoryl;
k) converting the compound of formula XI to a compound of formula XII, 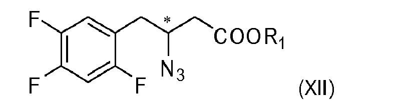 wherein * denotes a chiral atom and R₁ is C₁-C₆-alkyl
   by treatment with an azide reagent in an aprotic polar solvent or amide solvent with a complete or predominate conversion of chirality;
l) saponification of the compound of formula compound XII to a compound of formula compound XIII 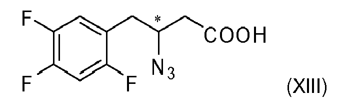 wherein * denotes a chiral atom;
m) amidation of the compound of formula compound XIII with the compound of formula VIII in the presence of coupling reagent in suitable organic solvent to form the compound of formula X.

In another optional embodiment (F) the compound of formula VIa obtained in step e) of the option (A) is further treated with amide coupling reagent, preferably selected from carbodiimides and O-benzylhydoxylamine optionally, in the presence of base to form a compound of formula VId, 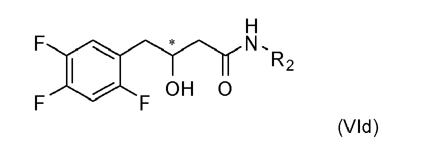 wherein * denotes a chiral atom and R₂ is selected from C₁-C₆-alkyl, aryl-C₁-C₄-alkyl, C₁-C₄-alkoxy, aryl-C₁-C₂-alkoxy, trifluoroacetyl, preferably C₁-C₄-alkoxy, aryl-C₁C₂-alkoxy, most preferably methoxy
and steps d) to h) of the option (A) are replaced with
n) sulfonation of the compound of formula VId to a compound of formula XId, 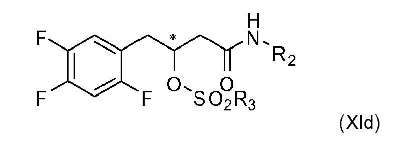 wherein * denotes a chiral atom and R₂ is selected from C₁-C₆-alkyl, aryl-C₁-C₄-alkyl, C₁-C₄-alkoxy, aryl-C₁-C₂-alkoxy, preferably methoxy or benzyloxy, and wherein R₃ is selected from methyl, ethyl, benzyl, p-toluenyl, p-bromobenzyl, p-nitrobenzyl and 10-camphoryl,
   by treatment with halogenides or anhydrides of a sulfonic acid in the presence of a weak base in an aprotic organic solvent;
o) cyclization of the compound of formula XId to a compound of formula XIV with a complete or predominate conversion of chirality, 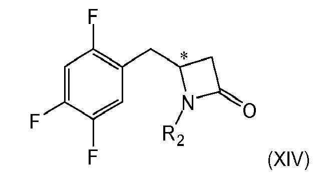 wherein * denotes a chiral atom and R₂ is same as above
   in the presence of strong base, preferably sodium hydride in a solvent selected from amides, halogenated alkanes or aromatic hydrocarbons;
p) hydrolysis of the compound of formula XIV to a compound of formula XV 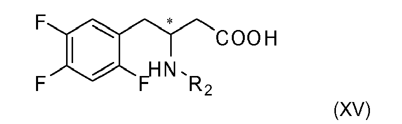 wherein * denotes a chiral atom
   by treatment with alkali or earth alkali hydroxide, preferably lithium hydroxide;
q) coupling of the compound of formula XV with the compound of formula VIII to form a compound of formula XVI 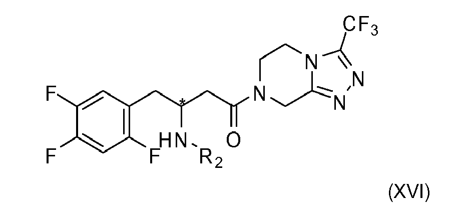 wherein * denotes a chiral atom
   in the presence of coupling reagents selected from pivaloyl chloride, chloroformates preferably isobutyl chloroformate and carbodiimides
r) eliminating of group R₂ by hydrolysis or reduction, preferably by catalytic hydrogenation.

In an alternative embodiment (G) a compound of structural formula IV obtained in step b) of the option (A), 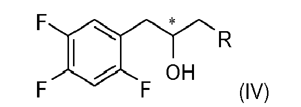 wherein wherein * denotes a chiral atom and R is an organometal inert group which could be conventionally converted to any one of cyano , aminocarbonyl, hydroxycarbonyl or alkoxycarbonyl group, defined as
R is 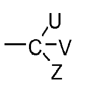 wherein U, V, and Z comprises
0-2 hydrogen atoms,
1-3 substituents selected from 1-oxa, 1-aza, 1-thia C₁-C₆-alkyl substituents in which two of them could be coupled to a 5-7-membered ring or two of them are joined to one and bound to carbon atom through a double bond,
C₁-C₂-alkylidene,
is converted to the compound of formula VI by reactions of hydrolysis alcoholysis, oxidation, or ozonization. Preferably the compounds of formula IV are selected from ortoesters (U, V, Z are trialkoxy), acetals (U, V, Z are dialkoxy or alkylenedioxy), thioacetals (U, V, Z are dialkylthio or alkylenedithio), aminals (U, V, Z are bis(dialkylamino or alkylenediamino)), or R is vinyl, optionally from esters (R is COOR₁, R₁ is C₁-C₆-alkyl) and amides (R is CONR₂R₃, R₁ is C₁-C₆-alkyl) if the reaction of compound of formula II is highly selected to epoxides and kinetically unfavoured to esters or amides.

In a special embodiment of the invention the method is used for the preparation of sitagliptin and related compounds and salts thereof, in which enantiomerically pure starting compounds are used. Hence in preferred embodiments the substituents in respective formulas have the following meaning
n is 3
X is fluoro in positions 2, 4 and 5 of benzene ring as regards to the side chain,
and the amide moiety of the carboxyl group is defined by the following formula III': 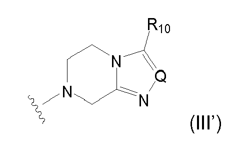 wherein Q is N, CH, C-CF₃, or C-phenyl, preferably N, and R₁₀ is H, C₁-C₄-alkyl or fluorinated C₁-C₂-alkyl, preferably trifluoromethyl, in particular by the following formula III'a (also denoted in the illustrative embodiments below as compound of formula 8):

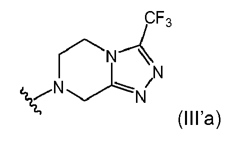

An illustrative and preferred but not limited approach of this special embodiment is shown in Scheme 5 and described in further detail as a representative embodiment below.

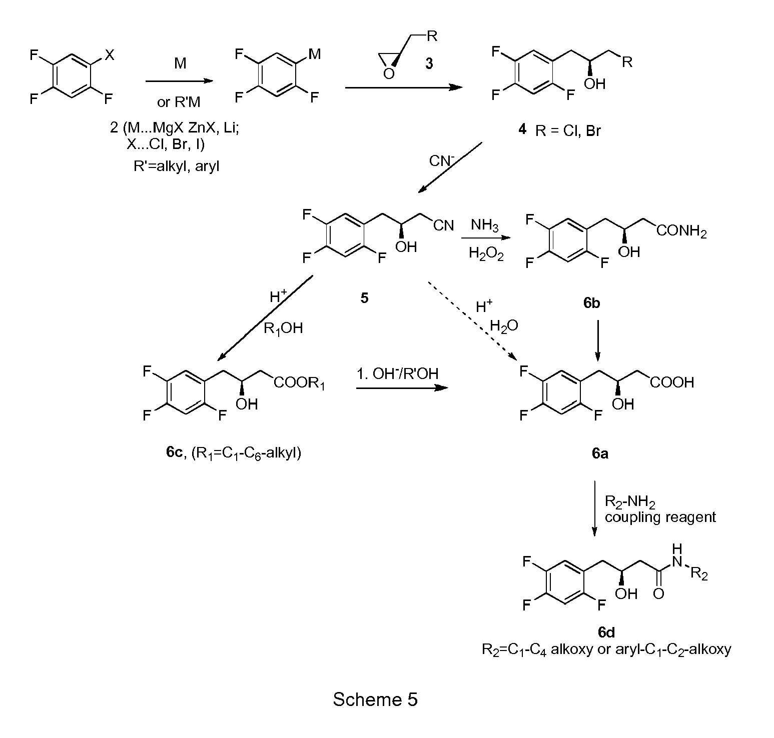

In the first step of the embodiment the (*S*)-epihalohydrin of formula **3** (compound of formula III, wherein * is in (S)-configuration and R is Cl or Br) reacts with 1-metalo-2,4,5-trifluorobenzene of formula **2** (compound of formula II, wherein M is MgX, ZnX, Li and X is Cl, Br, I) to form halohydrin of formula **4** ((S)-enantiomer of compound of formula IV, wherein X is CI or Br), optionally in the presence of catalytic amounts of copper (I) salts, preferably Cu₂X'₂ (X' is Cl, Br, I). This step comprises a one pot conversion of 1-halo-2,4,5-trifluorobenzene, preferably 1-bromo-2,4,5-trifluorobenzene via *"in situ"* produced 1-metalo-2,4,5-trifluorobenzene, formed by treating said compound with elemental metal, such as Mg, Zn or Li, or with alkyl or arylmetal reagents, such as Grignard reagents or alkyllithium to 1-halo substituted 1-(2-hydroxypropyl)-2,4,5-trifluorobenzenes (compound of formula **4**). Coupling of these organo-metalo species with epoxide proceeds faster in the presence of catalytic amounts of some transition metal salts such as manganese, cerium, copper salts, preferably copper (I) salts. For example 1-halomagnesio-2,4,5-trifluorobenzenes (compound of formula **2**, wherein M is MgCl) reacts efficiently with 1-1.3 equivalents of (S)-epichlorohydrin in the presence copper (I) chloride, copper (I) bromide or copper (I) iodide in a 0.001-1, preferably between 0.002-0.01 equivalent amount of compound of formula **2**. Best, but not limited solvents for the coupling reaction are ethers, preferably tetrahydrofurane and diethyl ether.

The next step of the embodiment comprises a conversion of halohydrin of formula **4** into cyanohydrin of formula **5** (compound of formula V) by reaction with cyanide anion, selected from alkali, earth alkali or quaternary ammonium salt, preferably from sodium or potassium cyanide. This reaction proceeds preferably in a protic solvent or in a mixture of protic and aprotic solvent. The preferable protic solvent is water, preferably in a mixture with aprotic polar solvent, most preferable medium is the water - N,N-dimethylformamide mixture. The conversion is suitably carried out at elevated temperatures above 50 °C, preferably at 70 °C to 150 °C most preferably at 80 °C to 100 °C for 1 h to 12 h, preferably 2 h to 5 h. The product is isolated by extraction into organic solvent, preferably selected from the group of aliphatic esters, aromatic hydrocarbons, ethers and haloalkanes.

The next step of the embodiment comprises may involve variant options. Hydrolysis of cyanohydrin of formula **5** leads to carboxylic derivatives of formula VI. The reaction may proceed in the presence of strong acids selected from hydrogen halogenides, preferably HCl, perchloric, alkanesulfonic, halo substituted alkanesulfonic, arylsulfonic acids, or sulfonic acids on polymer support in an C₁-C₆-alkanol, alone or diluted by a solvent. If the reaction is carried out without a solvent or in aqueous media the product is the corresponding acid **6a** ((S)-enantiomer of compound of formula VIa) and/or intermediate amide **6b** ((S)-enantiomer of compound of formula VIb).

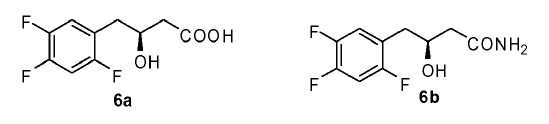

An alternative conversion of cyanohydrin of formula **5** to compound of formula **6a** in aqueous medium may also proceed in alkaline conditions, accelerated by addition hydrogen peroxide. Again compound of formula **6a** and compound of formula **6b** can be obtained but if time, the amount of peroxide and temperature are controlled, this conversion could be stopped at the stage of amide of formula **6b** formation.

In order to proceed more uniform reaction in milder conditions it is preferable to use alcohols as solvents, preferably C₁-C₆-alkanoles, most preferably ethanol or methanol, optionally diluted with water or aprotic organic solvent. The reaction of alcoholysis is carried out at temperatures between 0 °C and boiling point preferably between 0 °C and 60 °C for 0.5 h to 24 h preferably 6 h to 15 h to give a compound of formula **6c** ((*S*)-enantiomer of compound of formula VIc, wherein R₁ is C₁-C₆-alkyl). The product could be isolated by extraction into organic solvent and subsequent evaporation. In a most preferred example the compound of formula **4** is dissolved in ethanol followed by introducing gaseous hydrogen chloride. The reaction is carried out for 12 h, then ethanol is evaporated, addition of water follows, provoking a separation of the product as oil, which could be collected as it is, or extracted into organic solvent with subsequent removing of the solvent.

The next optional step of the embodiment comprises a saponification of the ester of formula **6c** treated by alkaline aqueous or alcoholic medium, preferably to obtain the acid of formula **6a**. Bases used for hydrolysis are selected from the group of alkali hydroxydes, earth-metal hydroxides, organic bases like amines, amidines and guanidines, preferably sodium hydroxide in methanol/water mixture.

The acid of formula **6a** may be isolated after acidification, optionally with previous diluting with water, followed by extraction with solvents, selected from the group of aliphatic esters, aromatic hydrocarbons, ethers and haloalkanes. If the conversion of cyanohydrin of formula **5** to compound of formula VI is not carried in alcoholic medium, the saponification of an ester may be omitted, or optionally achieved in order to hydrolyse the partial hydrolytic byproduct of formula **6b**.

In another approach compound of formula **5** is converted "*in situ*" into the ester of formula **6c** in an acidic alcoholic medium, followed by removing the majority of the volatile compound and hydrolysing the residue of not isolated ester of formula **6c** into the acid of formula **6a** by action of bases and/or acids. In this way is the compound of formula **6c** is provided in one pot process.

Alternatively, the next step of the embodiment comprises partial hydrolysis of the compound of formula **5** to the amide of formula **6b** in acidic or alkaline conditions with addition of hydrogen peroxide in water or C₁-C₆-alcohols or mixture thereof, preferably using sulfuric acid and hydrogen peroxide, or alkali metal hydroxides and hydrogen peroxide, most preferably the reaction is carried out with ammonia in 0.5 to 3 equivalent amount and hydrogen peroxide in 1 to 3 equivalent amount in an aqueous methanol or ethanol agitating at 0 °C to 70 °C, preferably at room temperature for 1 h to 12 h, more preferably for 1 h to 3 h. The reaction is carefully analyzed in order to prevent considerable conversion of the compound of formula to the acid of formula **6a**. The product is preferably isolated as a solid by filtration or centrifugation.

The next step of the embodiment comprises an amidation process with amines, selected from C₁-C₆-alkyl amines, aryl-C₁-C₄-alkylamines, C₁-C₄-alkoxy amines, aryl-C₁-C₂-alkoxyamines, trifluoroacetylamine, preferably O-benzylhydroxylamine in the presence of coupling reagents in a suitable organic solvent into compound of formula **6d**.

Common coupling reagents which are ordinary used in peptide chemistry are quite useful for this conversion, and they are suitably selected from pivaloyl chloride, chloroformates preferably isobutyl chloroformate, carbodiimides, e.g. N,N'-dicyclohexylcarbodiimide, N,N'-diisopropylcarbodiimide or N-ethyl-N'-(3-dimethyaminopropyl)carbodiimide, 1-hydroxybenztriazole, 1-methylpyridinium iodide, carbonyldiimidazole, optionally in the presence of organic tertiary amines. Preferable solvents for this reaction are selected from haloalkanes, nitriles, alkylcarboxylic esters, ethers, aromatic hydrocarbons.

An illustrative and preferred but not limited approach of this special embodiment is shown in Scheme 6 and described in further detail as a representative embodiment below.

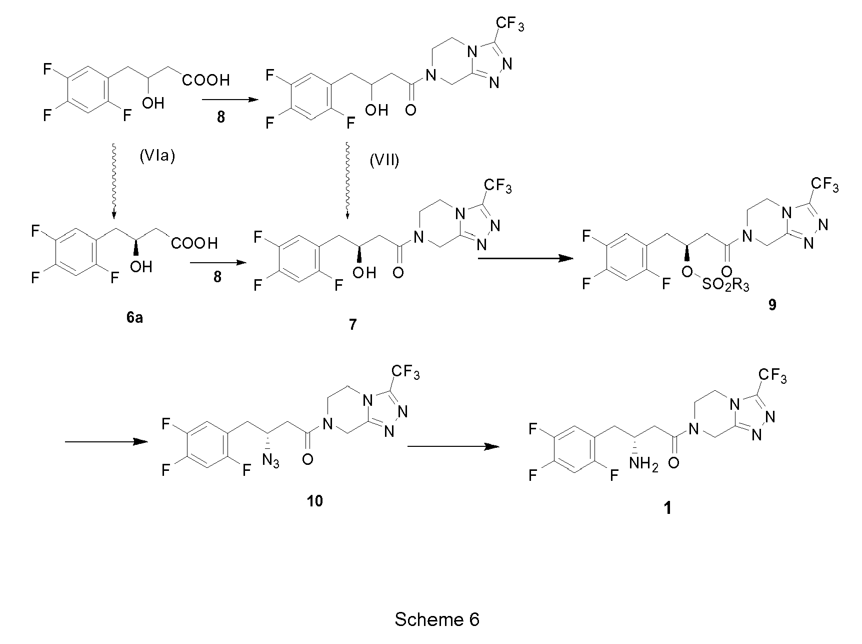

In this illustrative and preferred but not limited further part of the present embodiment, the compound of formula **6a** is converted to sitagliptin.

The first step of this embodiment comprises further treating of the compound of formula **6a** ((S)-enantiomer of a compound of formula VIa). This step comprises an amidation process with the heterocyclic moiety of formula **8** shown below into a compound of formula **7** in the presence of coupling reagent in a suitable organic solvent as defined above.

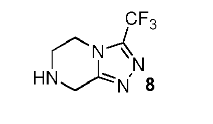

For the amidation process, common coupling reagents as well as solvents can be used like for the amidation process from the compound of formula **6a** to the compound of formula 6d as described above.

The compound of formula 7 is a suitable intermediate for preparation of sitagliptin. If the stereogenic center marked with an * is a mixture of (R)- and (*S*)-configuration a particular enantiomer might be separated off by chiral chromatography or by crystallization of stoichiometric (*R*)/(*S*) mixture with further recovering the excess enantiomer from mother liquor.

In the next step of this embodiment the compound of formula **7** is sulfonated to give the corresponding sulfonic esters of formula 9 in the presence of weak bases in an aprotic organic solvent (compound of formula IX, wherein R₃ is selected from methyl, ethyl, benzyl, p-methylbenzyl, p-bromobenyl, p-nitrobenzyl and camphoryl).

Agents used for sulfonation are suitably selected from halogenides or anhydrides of sulfonic acids, e.g. methanesulfonic, ethanesulfonic, benzenesulfonic, p-toluenesulfonic, p-bromobenzenesulfonic, p-nitrobenzenesulfonic, camphor-10-sulfonic. Suitable aprotic organic solvents are selected from carboxylic esters, haloalkanes, aromatic hydrocarbons, alkyl ethers, nitriles and N,N-dialkylamides. Suitably weak organic bases are selected from trialkylamines, dialkylarylamines or basic heterocycles e.g. imidazoles. Weak organic bases are preferred for this reaction, since strong bases provoke olefination of the product of formula **9**. In a most preferred but not limited case methanesulfonyl chloride as sulfonic halide and ethyldiisoproylamine as a weak base are used for this reaction. The product may be isolated after washing the reaction mixture with aqueous medium and subsequent evaporation of the solvent.

In the next step of this embodiment the compound of formula **9** is treated by an azide reagent, suitably selected from alkali metal azide or trialkylammonium azide in a solvent, preferably selected from aprotic polar solvents e.g. sulfolan, DMSO, or amide solvents, more preferably from formamide solvents, most preferably from a mixture of sulfolan and formamide or N-methylformamide at temperatures between 20 °C and 80 °C, preferably between 40 °C to 60 °C for 5 h to 48 h preferably for 12 h to 30 h to obtain the azido compound of formula **10**.

The reaction of azidation is the SN2 nucleophylic substitution, so the *(S)*-configuration on the chiral carbon atom is completely converted to (R). The *(R)*-configuration in formulas **10** and **1** as depicted in a form herein is the desired configuration for providing sitagliptin.

In the last step of this embodiment the compound of formula **10** is reduced, preferably by catalytic hydrogenation or reaction with trisubstituted phosphines in which the substituents may be equal or different, preferably selected from triphenylphosphine, trimethylphosphine or tributylphosphine.

An illustrative and preferred but not limited approach of this special embodiment is shown in Scheme 7 and described in further detail as a representative embodiment below. An alternative sequence of the invention comprises introduction of azido group into molecule before attaching heterocyclic moiety.

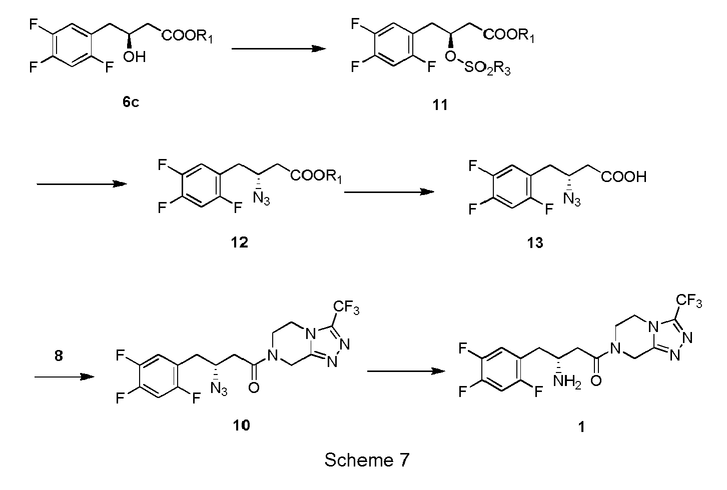

In the compound of formula **6c**, R₁ is C₁-C₆ alkyl, and in the compound of formula **11** R₃ is selected from methyl, ethyl, benzyl, p-methylbenzyl, p-bromobenzyl, p-nitrobenzyl and 10-camphoryl.

In the first step of this embodiment the compound of formula **6c** is sulfonated by halogenides or anhydrides of sulfonic acids as described above to give the corresponding sulfonic esters of formula **11** in presence of weak bases in an aprotic organic solvent. Weak organic bases as described above are used for this reaction, since strong bases provoke olefination of the product of formula **11**. In a most preferred but not limited case methanesulfonyl chloride as sulfonic halide and ethyldiisoproylamine as a weak base are used for this reaction. The product can be isolated after washing the reaction mixture with aqueous medium and subsequent evaporation of the solvent.

In the next step of this embodiment the compound of formula **11** is treated by an azide reagent selected from alkali metal azide or trialkylammonium azide in a solvent as described above to obtain the azido compound of formula **12**.

The reaction of azidation is the SN2 nucleophylic substitution, so the *(S)*-configuration on the chiral carbon atom is completely converted to *(R)*. The *(R)*-configuration in formulas **1, 10, 12, 13** as depicted in a form herein is the desired configuration for providing sitagliptin.

The next step of this embodiment comprises a saponification of the azidoester of formula **12**, wherein R₁ is C₁-C₆-alkyl treated by alkaline aqueous or alcoholic medium, to obtain an acid of formula **13**. Bases used for hydrolysis are selected from the group of alkali hydroxydes, earth-metal hydroxides, organic bases like amines, amidines and guanidines, preferably sodium hydroxide in methanol/water mixture.

The next step of this embodiment comprises an amidation process with the heterocyclic moiety of formula 8 into the compound of formula **13** in the presence of coupling reagent in a suitable organic solvent as defined above. For the amidation process, common coupling reagents as well as solvents can be used like for the amidation process from the compound of formula **6a** to the compound of formula **6d** described above.

In the last step of this embodiment the compound of formula **10** is reduced, preferably by catalytic hydrogenation or by reaction with trisubstituted phosphines in which the substituents may be equal or different, preferably selected from triphenylphosphine, trimethylphosphine or tributylphosphine to give sitagliptin.

An illustrative and preferred but not limited approach of this special embodiment is shown in Scheme 8 and described in further detail as a representative embodiment below.

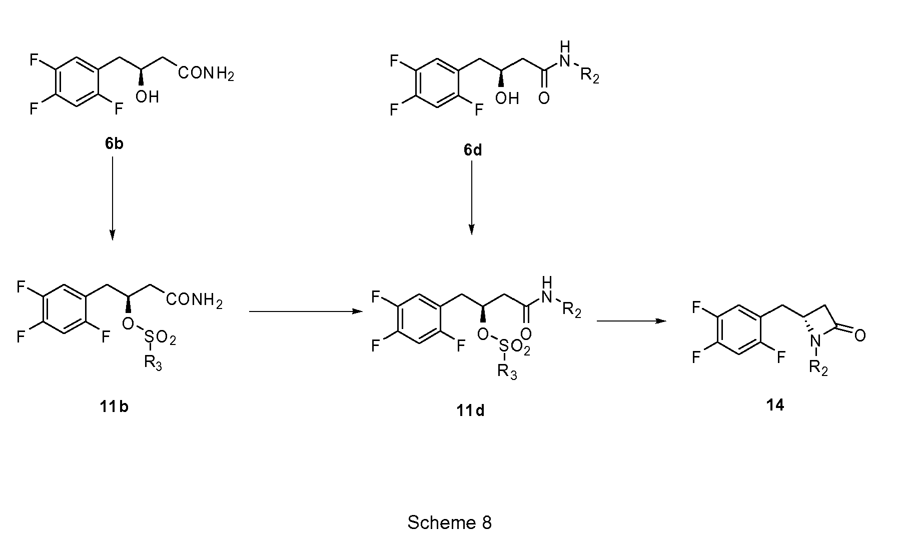

In another embodiment the compound of formula **6b** is sulfonated by halogenides or anhydrides of sulfonic acids as defined above to give the corresponding sulfonic esters of formula **11b** in presence of weak bases in an aprotic organic solvent. Weak organic bases as defined above are used for this reaction, since strong bases provoke olefination of the product of formula **11b.** In a most preferred but not limited case methanesulfonyl chloride as sulfonic halide and ethyldiisopropylamine as a weak base are used for this reaction. The reaction is preferably carried out in dichloromethane at 0 °C to 20 °C in the presence of 1-1.5 equivalents of methanesulfonyl chloride and 1-2 equivalents of base for 0.5 h to 6 h. The product is isolated after washing the reaction mixture with aqueous medium and subsequent evaporation of the solvent.

In an alternative the amide of formula **11b** can be substituted with a halogenide R₂-X, wherein R₂ is C₁-C₆-alkyl, aryl-C₁-C₄-alkyl and trifluoroacetyl in order to obtain a compound with R₂ which activates the system to cyclisize to azetidone of formula **14** in basic conditions, preferably R₂ is trifluoroacetyl.

An illustrative and preferred but not limited approach of this special embodiment is shown in Scheme 9 and described in further detail as a representative embodiment below.

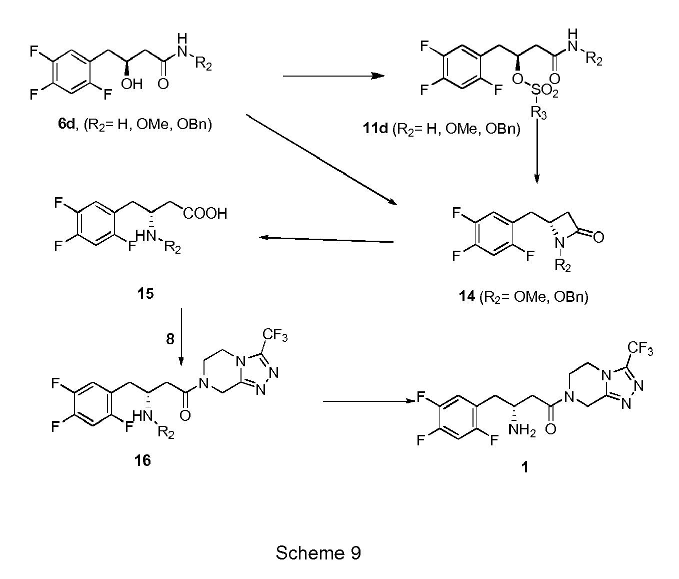

In the compound of formula 11d R₃ is selected from methyl, ethyl, benzyl, p-methylbenzyl, p-bromobenyl, p-nitrobenzyl and camphoryl.

In the first step of this embodiment the compound of formula **6d**, wherein R₂ is selected from H, OMe and OBn, is sulfonated by halogenides or anhydrides of sulfonic acids as defined above to give the corresponding sulfonic esters of formula **11d** in presence of weak bases in an aprotic organic solvent. Weak organic bases as defined above are used for this reaction, since strong bases provoke olefination of the product of formula **11d.** In a most preferred but not limited case methanesulfonyl chloride as sulfonic halide and ethyldiisopropylamine as a weak base are used for this reaction. The reaction is preferably carried out in dichloromethane at 0 °C to 20 °C in the presence of 1-1.5 equivalents of methanesulfonyl chloride and 1-2 equivalents of base for 0.5 h to 6 h. The product is isolated after washing the reaction mixture with aqueous medium and subsequent evaporation of the solvent.

The next step of this embodiment comprises the conversion of oxyamide of formula **11d,** wherein R₂ is selected from a group of OMe or OBn to the azetidinone of formula **14** in the presence strong base, preferably sodium hydride in a solvent selected from amides, halogenated alkanes or aromatic hydrocarbons. Alternatively, the same azetidinone can be prepared by Mitsunobu reaction using azodicarboxylate or diisopropyl azodicarboxylate in the presence of phosphines, preferably triphenylphosphine in one step directly from the oxyamide **6d,** wherein R₂ is selected from a group of OMe or OBn.

In the next step of this embodiment the azetidinone of formula **14**, wherein R₂ is OMe or OBn, is further cleaved by alkaline hydrolysis using alkali or earth alkali hydroxide, preferably lithium hydroxide to give β-oxyamino acids of formula **15,** which are further coupled with heterocyclic moiety **8** into compound of formula **16** in the presence of coupling reagent in suitable organic solvent as described above.

For the amidation process, common coupling reagents as well as solvents can be used like for the amidation process from **6a** to **6d** described above.

The next step of this embodiment comprises the reduction of β-oxyamino compounds of formula **16**, wherein R₂ is OMe or OBn, to sitagliptin of formula 1 by common methods such as catalytic hydrogenation, reduction by alkali metal in ammonia, preferably lithium or sodium or reduction by reducing elemental metal in an acid, preferably zinc or tin in hydrochloric acid.

The *(R)*-configuration in formulas **14, 15, 16** and **1** as depicted in a form herein is the desired configuration for providing sitagliptin.

While preferred embodiments have been described, it will become apparent that variations are possibly and are contemplated herein, to yield sitagliptin or analogues thereof, or other gliptins or other β-amino acids and β-amino acids derivatives, when starting with compounds of formula II and III to obtain the compound of formula IV with suitable subsequent synthesis steps.

The following examples illustrate the present invention and are not intended to limit the scope of the invention.

### Experimental procedures

### Example 1: Preparation of (S)-1-chloro-3-(2,4,5-trifluorophenyl)propan-2-ol (formula 4)

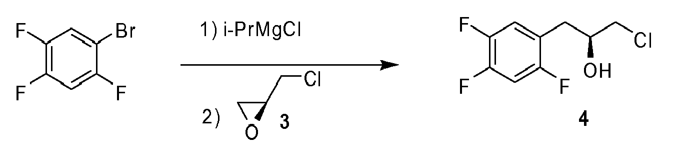

### Preparation of 2,4,5-trifluorophenylmagnesium chloride solution:

700 mL of tetrahydrofuran was charged into reactor and cooled to 0 °C, followed by the consecutive addition of 350 mL 2 M i-propylmagnesium chloride and 69 mL 1-bromo-2,4,5-trifluorobenzene applying stirring and maintaining temperature of the reaction mixture between 0 °C and 5 °C. After the addition, the reaction mixture was stirred gently for 1 h at 0 °C to 5 °C and for 3 h at 22 °C obtaining the solution of 2,4,5-trifluorophenylmagnesium chloride.

### Determination of activity of the obtained solution:

0.25 g of iodine was weighed into small reaction flask and titrated with the solution obtained. When darkly brown solution turned its color to white suspension, the consumption of solution was ready. Typical consumption was about 1.85 mL/mmol of iodine.

### Conversion to compound of formula 4:

To the whole amount of above prepared solution of Grignard reagent 28 mL of (S)-epichlorohidrin (formula 3) was added during stirring, followed by the addition of 0.80 g copper(I) chloride. Exotermic reaction started and when inner temperature raised to 40 °C, external cooling was applied, lowering the internal temperature to 25 °C. 28 mL of (S)-epichlorohidrin divided into two equal portions were added.

After the addition, the reaction mixture was stirred for 5 h at 25 °C, followed by the addition of 75 mL acetic acid and 750 mL methyl tert-butyl ether. The resulted mixture was washed consecutively with 700 mL water; mixture of 700 mL water, 80 mL brine, 80 mL 20% ammonium chloride, and 750 mL 2% sodium bicarbonate.

Organic phase was evaporated at 60 °C and 5 mbar, to yield 131 g of crude product of compound of formula **4** in the form of yellowish oil.

¹H NMR (DMSO-d₆): δ 2.64 (dd, 1H), 2.83 (dd, 1H), 3.49-3.64 (m, 2H), 3.85 3.90 (m,1H), 5.31 (d,1H), 7.37-7.48 (m, 2H).

### Example 2: Preparation of (S)-3-hydroxy-4-(2,4,5-trifluorophenyl)butyronitrile (formula 5)

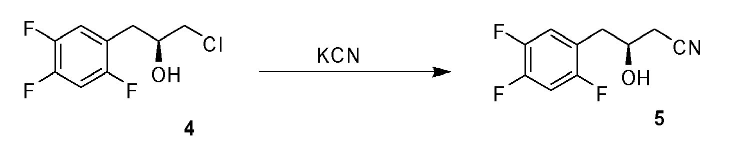

128 g compound of formula **4**, 60 g potassium cyanide, 7 g sodium iodide, 160 mL N,N-dimethylformamide and 220 mL water was charged into reactor. The mixture was heated for 3 h at 95 °C during stirring, forming homogeneous solution. After cooling to 24 °C, the reaction mixture was washed with 340 mL n-heptane. Water phase was extracted twice with 1300 mL and 400 mL ethyl acetate. Combined organic phases were washed consecutively with 2 x 950 mL water and 2 x 1100 mL of diluted brine (950 mL water and 150 mL brine).

Organic phase was evaporated at 60 °C and 5 mbar, the residue was dissolved in 150 mL ethyl acetate and evaporated again at 60 °C and 5 mbar to remove water to yield 102.3 g of clear, brown syrupy product of compound of formula **5**.

¹H NMR (DMSO-d₆): δ2.52 (dd, 1H), 2.65-2.79 (m, 3H), 3.98 (s/wide/, 1H), 5.51 (d,1H), 7.37-7.46 (m, 2H).

### Example 3: Preparation of ethyl (S)-3-hydroxy-4-(2,4,5-trifluorophenyl)butyrate (formula 6)

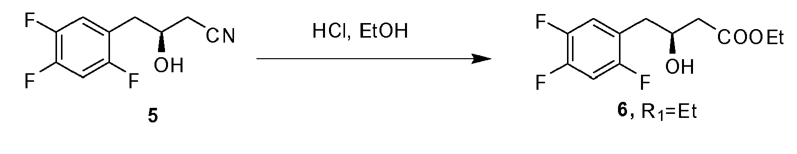

45 g of hydrogen chloride gas was dissolved in 100 mL absolute ethanol at -10 °C. 75 g of compound of formula 5 was added during stirring. The temperature of reaction mixture slowly raised to 35 °C, then it was cooled to 23 °C and aged for 8 h at the same temperature. Afterwards 4 g of active charcoal and 80 mL of ethanol were added, followed by stirring at 60 °C for 10 min and subsequent filtering off the charcoal.

The filtrate was subjected to evaporation at 58 °C and 35 mbar. To the oily residue obtained 180 mL of water were added and the resulted solution was stirred at 40 °C for 2 h and biphasic mixture was formed, which was finally extracted twice with 180 mL of ethyl acetate. Combined extracts were washed with 130 mL of diluted brine (110 mL water and 20 mL brine). Organic phase was evaporated at 60 °C and 5 mbar. The obtained oily residue was dissolved in 150 mL ethyl acetate and evaporated to remove water as much as possible.

The residue was dissolved in 300 mL ethyl acetate. The resulting solution was dried for 3 h with 20 g of anhydrous sodium sulfate. Drying agent was filtered off and the obtained filtrate was evaporated at 60 °C and 4 mbar to yield 102.5 g of clear brown oily product of compound of formula **6**.

¹H NMR (DMSO-d₆): δ 1,15 (t, 3H), 2.34 (dd, 1H), 2.34 (dd, 1H), 2.66 (dd, 1H), 2.72 (dd, 1H), 4.02(q,2H), 4.06 4.10 (m, 1H), 5.02(d,1H), 7.35-7.44 (m, 2H).

### Example 4: Preparation of (S)-3-hydroxy-4-(2,4,5-trifluorophenyl)butyric acid (formula 6a)

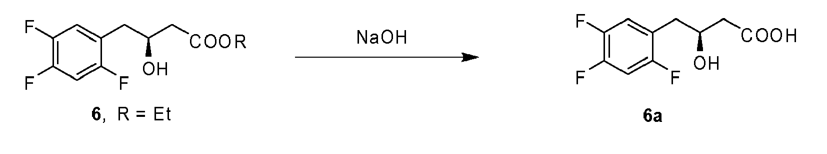

93.0 g of compound of formula **6** (R₁ = Et) was dissolved in the mixture of 280 mL THF and 700 mL water at 40 °C. During stirring, 49 mL of 8 M aqueous sodium hydroxide were poured gradually within 10 min. The reaction mixture was kept stirring for 2 h at 40 °C, followed by washing the reaction mixture with 900 mL of methylcyclohexane. To the aqueous phase 930 mL of ethyl acetate, 230 mL of water and 75 mL 6 N aqueous hydrochloric acid were added.

After vigorous stirring, organic phase was separated and washed with 700 mL of diluted brine (570 mL of water and 130 mL salt). Organic phase wais subjected to evaporation at 55 °C and 5 mbar, obtaining 80.1 g of crude solid product of compound of formula **6a**, which could be recrystallised from toluene (190 mL) and obtaining 62 g of pure solid.

¹H NMR (DMSO-d₆): δ 2.26 (dd, 1H), 2.34 (dd, 1H), 2.64 (dd, 1H), 2.72 (dd, 1H), 4.02 4.09 (m, 1H), 4.92(s/wide/,1H), 7.34-7.46 (m, 2H), 12.06(s/wide/,1H).

### Example 5: Preparation of 7-((S)-3-hydroxy-4-(2,4,5-trifluorophenyl)butyryl)-(3-(trifluoromethyl)-5,6,7,8-tetrahydro-[1,2,4]triazolo[4,3-a]pyrazine (formula 7)

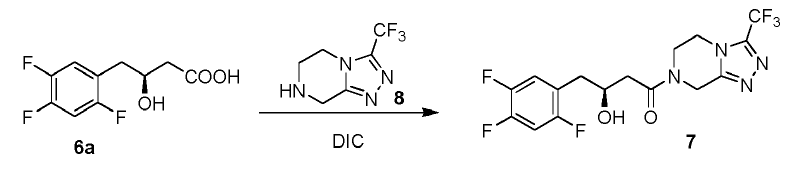

35 g of compound of formula **6a** and 28.3 g of compound of formula **8** were dissolved in 364 mL dichloromethane, forming clear solution, which was pre-cooled to 16 °C before adding of 23.5 mL N,N'-diisopropylcarbodiimide. During gradual addition (15 min) of carbodiimide, the temperature was maintained between 20 °C to 25 °C applying external cooling. After the addition, the reaction mixture was stirred at 23 °C for 3 h and then aged 3 h at 0 °C, forming white suspension of precipitated N,N'-diisopropylurea. Suspension was filtered and the precipitate was washed with 2 x 50 mL dichloromethane. Clear, yellowish-brown filtrate was washed with 360 mL of diluted hydrochloric acid (obtained from 350 mL of water and 7.0 mL of 6 N HCl), 380 mL 0.5 % aqueous sodium bicarbonate, and 350 mL water.

Organic phase was evaporated at 50 °C and 2 mbar, obtaining 58.4 g of clear, pale-brownish amber-like product of compound of formula **7**, which solidified on standing.

¹H NMR (DMSO-d₆): δ 2.45-2.48(m, 1H), 2.65-2.78 (m,3H), 3.53-3.55(m, 2H), 3.96-4.21 (m,3H), 4.85-5.05(m,2H), 7.35-7.40 (m, 2H).

### Example 6: Preparation of 7-((S)-3-methanesulfonyloxy-4-(2,4,5-trifluorophenyl)butyryl)-(3-(trifluoro methyl)-5,6,7,8-tetrahydro-[1,2,4]triazolo[4,3-a]pyrazine (formula 9)

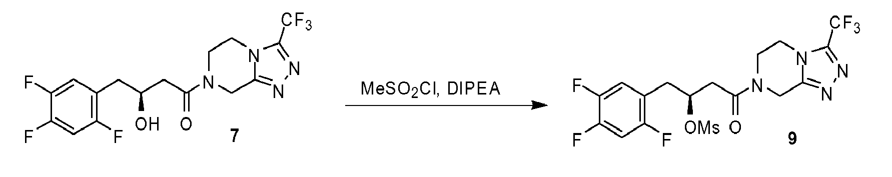

13.4 g of compound of formula **7** was dissolved in 70 mL of dichloromethane, followed by the addition of 15 mL of ethyldiisopropylamine. The turbid solution was cooled to 15 °C during stirring. During gradual addition of 4.7 mL of methanesulfonyl chloride the temperature of reaction mixture was maintained between 15 °C to 20 °C. After addition, stirring was continued for 1 h at 23 °C. Reaction mixture was then diluted with 70 mL of dichloromethane and consecutively washed with 100 mL 5% aqueous sodium bicarbonate and twice with 110 mL of the mixture prepared by mixing 100 mL water and 10 mL brine.

Evaporation of the reaction mixture at 5 mbar and 55 °C gave 16.1 g of yellowish-brown, solid foamy product of compound of formula **9**.

¹H NMR (DMSO-d₆): δ 2.80-3.12(m, 7H), 3.89-4.0(m,2H), 4.03-4.27 (m, 2H), 4.80-4.95(m,2H), 5.12-5.20(m,1H), 7.38-7.46 (m, 2H).

### Example 7: Preparation of 7-((R)-3-azido-4-(2,4,5-trifluorophenyl)butyryl)-(3-(trifluoromethyl)-5,6,7,8-tetrahydro-[1,2,4]triazolo[4,3-a]pyrazine (formula 10)

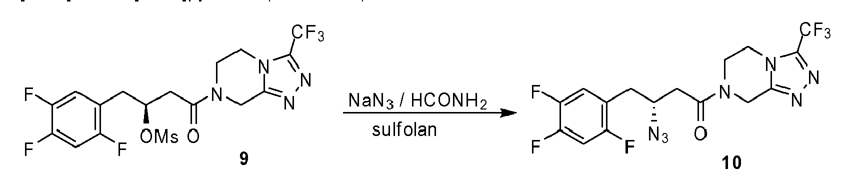

8.0 g of compound of formula **9** was dissolved in the mixture of 24 mL formamide and 14.0 g sulfolane and 3.0 g of sodium azide are added. The mixture was stirred at 50 °C for 24 h, then cooled to 24 °C and 70 mL of ethyl acetate was added. The suspension formed was washed consecutively by 3 x 80 mL water. The organic layer was evaporated at 52 °C and 4 mbar, yielding 7.2 g of brown amber-like, crude product of compound of formula **10**.

NMR (DMSO-d₆): δ 2.52-2.70(m,2H), 2.80-2.93(m,2H), 3.91-4.31(m,5H), 4.89-5.13(m,2H), 6.90-6-96(m,1H), 7.09-717(m,1H);

IR: 2123 cm-1 (N3), 1645 cm-1 (N-C=O).

### Example 8: Preparation of 7-((R)-3-amino-4-(2,4,5-trifluorophenyl)butyryl)-(3-(trifluoromethyl)-5,6,7,8-tetrahydro-[1,2,4]triazolo[4,3-a]pyrazine (formula 1, sitagliptin)

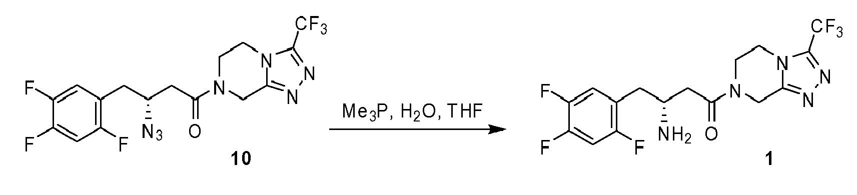

12.1 g of crude compound of formula 9 from Example 7 was dissolved in the mixture of 12 mL tetrahydrofuran, 12 mL ethanol, 2 mL acetic acid and 0.6 mL water. During gradual addition of 34.3 mL 1 M solution of trimethylphosphine in tetrahydrofurane the temperature of exothermic reaction mixture was maintained bellow 30 °C by external cooling. After the addition, reaction mixture was stirred at 23 °C for 16 h and evaporated, providing 15.5 g of residue.

The residue was dissolved in 30 mL ethyl acetate. The product was taken into the aqueous phase by adding of mixture of 60 mL of water and 3.5 g 85% H₂SO₄. Aqueous phase was washed twice with 2 x 40 mL i-propyl acetate and alkalised to pH of 9 with 11 mL 7 M KOH. The product was extracted with 1 x 60 mL ethyl acetate and 2 x 30 mL ethyl acetate. Combined organic phases were washed with the mixture of 30 mL water and 5 mL brine and evaporated at 50 °C and 5 mbar, yielding 7.03 g of the syrupy product of compound of formula 1 in the form of free base, which solidified on standing.

### Conversion of free base to sitagliptin phosphate:

0.4 g of the free base of compound of formula 1 was dissolved in 4 mL of ethanol followed by the addition of 0.2 g 85% orto-phosphoric acid. The resulting suspension was cooled for 15 min at 0 °C and filtered. The product was washed with 2 mL of ethanol and 4 mL of ether and dried on filter for 1 h. 0.26 g of the off-white powdery product of sitagliptin phosphate was obtained.

### Example 9: Preparation of ethyl (S)-3-methansulfonyloxy-4-(2,4,5-trifluorophenyl)butyrate (formula 11)

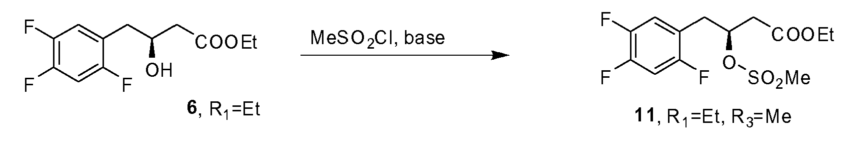

68 g of compound of formula **6** (Rᵢ = Et) was dissolved in the mixture of 340 mL dichloromethane and 73 mL ethyldiisopropylamine at 23 °C. Afterwards, the solution was cooled at 2 °C to 4 °C, following by addition of 25.3 mL of methanesulfonyl chloride while stirring and maintaining the temperature between 5 °C to 10 °C. The reaction mixture was then stirred for 1.5 h at 21 °C, finally diluted with additional 340 mL dichloromethane and washed consecutively with 500 mL of 5% aqueous sodium bicarbonate, and twice with 550 mL of diluted brine (500 mL water and 50 mL brine). Organic phase was evaporated at 60 °C and 3 mbar, yielding 87.3 g of brown, thick oil of compound of formula **11**.

¹H NMR (DMSO-d₆): δ 1.13(t,1H), 2.69(dd,1H), 2.77(dd,1H), 3.03(s,3H), 3.03(m,2H), 4.07(dq,2H), 5.10-5.16(m,1H), 7.41-7.66(m,2H).

### Example 10: Preparation of ethyl (R)-3-azido-4-(2,4,5-trifluorophenyl)butyrate (formula 12)

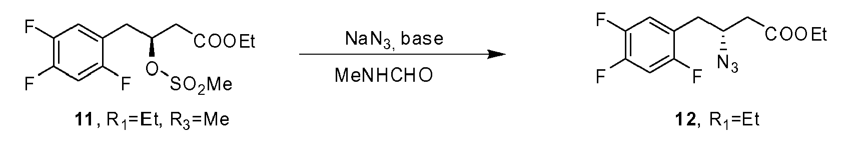

10.0 g of compound of formula **11** (R₁ = Et, R₃ = Me) was dissolved in the mixture of 30 mL N-methylformamide and 24.0 g sulfolane, then 4.8 g of sodium azide were added. The mixture was stirred at 50 °C for 7 h, then cooled to 24 °C, and finally 90 mL of ethyl acetate were added. The suspension formed was washed consecutively with 3 x 100 mL water. The organic layer was evaporated at 52 °C and 4 mbar, yielding 7.2 g of crude oily product of compound of formula **12** (R₁ = Et), which contained about 20% of olefinic side product.

¹H NMR (DMSO-d₆): δ 1.29(t,3H), 2.52-2.54(m,2H), 2.79(dd,1H), 2.90(dd,1H), 4.06-4.10(m,1H), 4.19(q,2H), 6.88-6.98(m,2H).

IR: 2120 cm⁻¹ (N₃), 1737 cm⁻¹ (O-C=O)

### Example 11: Preparation of (S)-3-hydroxy-4-(2,4,5-trifluorophenyl)butyramide (formula 6b)

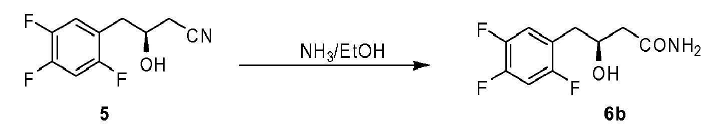

10.0 g of compound of formula **5** was dissolved in the mixture of 5 mL of ethanol and 5 mL 25% aqueous ammonia. During stirring, 16 mL of 30% aqueous hydrogen peroxide divided in three equal portions were added gradually in 20 min long intervals. The addition of peroxide was accompanied with foaming and spontaneous heating of reaction mixture. After the addition the reaction mixture was stirred for 2 h at 23 °C and 25 mL of water were added. The suspension formed was aged at 0 °C to 5 °C for 20 min, followed by filtering-off the off-white solid product which was washed with 8 mL cold water and air-dried on filter for 2 h. After additional drying in vacuum-dessicator at 80 °C and 5 mbar 8.3 g of the compound of formula **6b** was collected.

¹H NMR (DMSO-d₆): δ 2.11-2.20(m,2H), 2.60(dd,1H), 2.70(dd,1H), 4.04(m,1H), 4.91(s,1H), 6.82(s,1H), 7.29(s,1H), 7.35-7.46(m,2H).

### Example 12: Preparation of (S)-3-methanesulfonyloxy-4-(2,4,5-trifluorophenyl)butyramide (formula 11b)

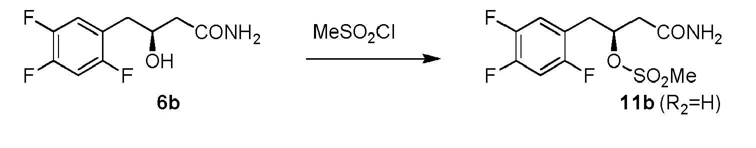

12.0 g of compound of formula **6b** was suspended in the solution of ethyldiisopropylamine in 105 mL dichloromethane. While vigorous stirred 4.5 mL of methanesulfonyl chloride were introduced gradually maintaining the temperature of reaction mixture between 10 °C to 14 °C applying external cooling. After the addition stirring was continued for 1 h at 22 °C, during which the suspension turned to bright, reddish-brown, clear solution. The reaction mixture was washed twice with 90 mL of water, followed by evaporation at final temperature 52 °C and 5 mbar of final pressure to yield 13.5 g of brownish-white solid product of compound of formula **11b.**

¹H NMR (DMSO-d₆): δ 2.42-2.52(m,2H), 2.96-3.09(m,5H), 3.01 (s), 5.09-5.15(m,1H), 7.04(s,1H), 7.39-7.54(m,3H).

### Example 13: Preparation of (S)-3-hydroxy-N-methoxy-4-(2,4,5-trifluorophenyl)butanamide (formula 6d, R=OMe)

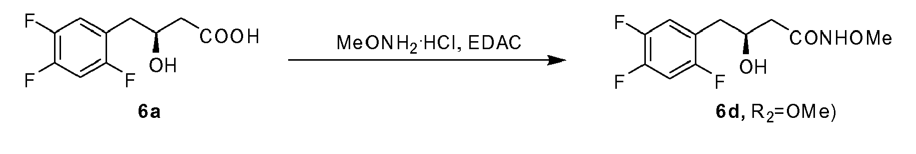

30.0 g of compound of formula **6a,** 3.23 g of 1-hydroxybenzotriazol hydrate (HOBt) and 13.7 g methoxyamine hydrochloride were suspended in 230 mL of water and 5.25 g of lithium hydroxide hydrate were added. After 10 minutes of stirring at 23 °C, 15.3 g of N-ethyl-N-(3-dimethylaminopropyl)carbodiimide hydrochloride (EDAC) were added, maintaining the temperature at 23 °C. After 30 minutes another portion of 5.25 g lithium hydroxide and 15.3 g of EDAC were added in the same manner as before. After 2 hours, 2.25 g of EDAC were added, keeping stirring for another 1.5 hour, then the reaction mixture was acidified with 6N HCl to pH 3. The white suspension was stirred 30 minutes at 0 °C, filtered and washed with 70 mL of water. The wet product was suspended in the mixture of 90 mL water and 5 mL of 25% aqueous ammonia. The suspension was thoroughly stirred 10 minutes, followed by filtration and washings with 50 mL of water. After drying in vacuum dessicator at 70 °C and 20 mbar for 18 hours, 30.3 g of the product in the form of white powder were obtained.

¹H NMR (DMSO-d₆): δ 1.98-2.08 (m, 2H), 2.61 (dd, 1H), 2.71 (dd, 1H), 3.55 (s, 3H), 4.05 (m, 1H), 4.96 (d, 1H), 7.36-7.48 (m, 2H), 10.95 (s, 1H).

### Example 14: Preparation of (S)-N-(benzyloxy)-3-hydroxy-4-(2,4,5-trifluorophenyl)butanamide (6d, R₂ = OBn)

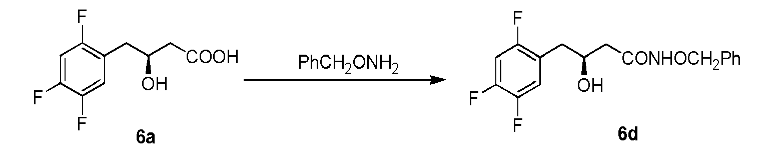

15.0 g (*S*)-3-hydroxy-4-(2,4,5-trifluorophenyl)butanoic acid (**6a**), 10.8 g O-benzylhydroxylamine hydrochloride, 105 mL dichloromethane and 12 mL ethyldiisopropylamine were charged in reaction flask. During stirring at 2 °C to 4 °C, 11.1 mL of N,N'-diisopropylcarbodiimide were added gradually in 10 minutes. After the addition the reaction mixture was stirred for 16 hours at 23 °C, followed by cooling at 2 °C for 20 minutes. The white precipitate (urea derivative) was filtered-off and discarded. The filtrate was washed two times with mixture of 60 mL water and 15 mL of brine, followed by evaporation at 50 °C and 5 mbar, providing 26.0 g of crude product (**6d**).

¹H NMR (DMSO-d₆): δ 2.08(d,2H), 2.59(dd,1H), 2.69(dd,1H), 4.03-4.06(m,1H), 4.75(s,2H), 7.29-7.42(m,7H).

### Example 15: Preparation of (S)-4-(methoxyamino)-4-oxo-1-(2,4,5-trifluorophenyl)butan-2-yl methane-sulfonate (11d, R₂=OMe)

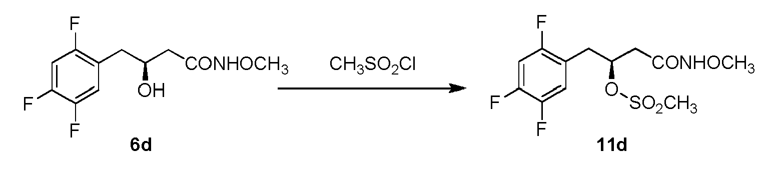

To the solution of 8.8 g of the compound of formula **6d** in 80 mL of dichloromethane at -20 °C 2.8 mL methanesulfonyl chloride were added, followed by gradual addition of 7.2 mL ethyldiisopropylamine, maintaining the temperature at -20 °C. After the addition the reaction mixture was stirred for 1 hour at 0 °C, followed by washings with the mixture of 80 mL water and 8 mL 2 M H₃PO₄, and 2 x (60 mL water + 5 mL brine). Organic phase was evaporated at 55 °C and 5 mbar of final pressure, yielding 7.75 g of crude product **(11d)**.

¹H NMR (DMSO-d₆): δ 2.32(dd,2H), 2.41 (dd,2H), 3.01 (s,3H), 2.99-3.10(m,2H), 3.56(s,3H), 5.08-5.16(m,1H), 7.41-7.56(m,2H), 11.20(s,1H).

### Example 16: Preparation of (S)-4-(methoxyamino)-4-oxo-1-(2,4,5-trifluorophenyl)butan-2-yl methane-sulfonate (11d, R₂ = OMe)

2.6 g of the compound of formula **6d** from previous experiment, 12 mL dichloromethane and 0.63 mL methanesulfonyl chloride are charged in the reaction flask and cooled to 0 °C. During stirring at 0 °C 1.5 mL sym-collidine was added gradually, maintaining the temperature at 0 °C. After the addition the reaction mixture was stirred for 1 hour at 20°C, followed by washing the reaction mixture 1 x (30 mL water and 3 mL brine), 1 x (30 mL water and 10 mL H₃PO₄) and 1 x (30 mL water and 3 mL brine). The organic phase was evaporated at 55 °C and 5 mbar of final pressure, obtaining 3.15 g of crude product **(11d)** in the form of clear brownish amber with similar spectroscopic data as in Example 15.

### Example 17: Preparation of (S)-4-(benzyloxyamino)-4-oxo-1-(2,4,5-trifluorophenyl)butan-2-yl methanesulfonate (11d, R₂ = OBn)

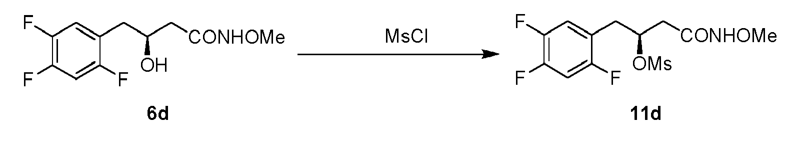

2.6 g of the compound of formula **6d** from Experiment 16, 12 mL dichloromethane and 0.63 mL methanesulfonyl chloride are charged in the reaction flask and cooled to 0 °C. During stirring at 0 °C 1.5 mL sym-collidine was added gradually, maintaining the temperature at 0 °C. After the addition the reaction mixture was stirred for 1 hour at 20°C, followed by washing the reaction mixture 1 x (30 mL water and 3 mL brine), 1 x (30 mL water and 10 mL H₃PO₄) and 1 x (30 mL water and 3 mL brine). The organic phase was evaporated at 55 °C and 5 mbar of final pressure, obtaining 3.15 g of crude product **(11d)** in the form of clear brownish amber.

¹H NMR (DMSO-d₆): 2.49-2.81 (m,4H), 3.35(s,3H), 4.15-4.18(m,1H), 5.08(s,2H), 7.35-7.45(m,7H).

### Example 18: Preparation of (S)-4-(benzyloxyamino)-4-oxo-1-(2,4,5-trifluorophenyl)butan-2-yl methane-sulfonate (11d, R = OBn)

2.6 g of the compound (**6d**), 10 ml dichloromethane and 0.50 ml methanesulfonyl chloride are charged in the reaction flask and cooled to 0 °C. During stirring at 0 °C 1.2 ml sym-collidine was added gradually, maintaining the temperature at 0 °C. After the addition the reaction mixture was stirred 1 hour at 20 °C, followed by washing the reaction mixture 1 x (25 ml water and 2 ml brine), 1 x (25 ml water and 8 ml H₃PO₄) and 1 x (25 ml water and 2 ml brine). The organic phase was evaporated at 52 °C and 7 mbar of final pressure, obtaining 2.93 g of crude amber-like product **(11d)** - ([M+H]+ = 418).

### Example 19: Preparation of (R)-1-methoxy-4-(2,4,5-trifluorobenzyl)azetidin-2-one (14, R₂=OMe)

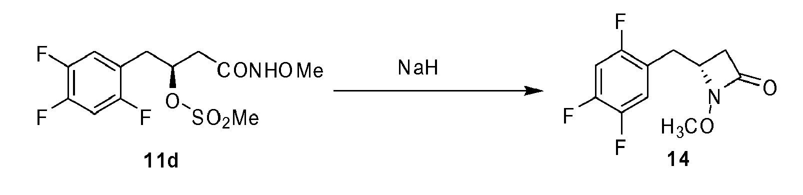

To the solution of 2.2 g of the compound of formula **6d** in the mixture of 16.0 mL dichloromethane and 4.0 mL N,N-dimethylformamide at 0 °C 0.29 g 60% sodium hydride was added gradually, keeping foaming under control. After the addition, the reaction mixture was kept stirring 4 hours at 23 °C, followed by the addition of 0.26 mL acetic acid and evaporation at 35 °C and 25 mbar. The residue is suspended in 50 mL of diethyl ether, followed by washing with 30 mL of water. The water phase is re-extracted with 30 mL of diethyl ether. Combined ether phases were washed with 2 x (20 mL water + 2 mL brine) and evaporated at 50 °C and 5 mbar of final pressure, obtaining 1.60 g of the crude product in the form of brownish syrup.

### Example 20: Preparation of (R)-1-methoxy-4-(2,4,5-trifluorobenzyl)azetidin-2-one (formula 14, R₂=OMe)

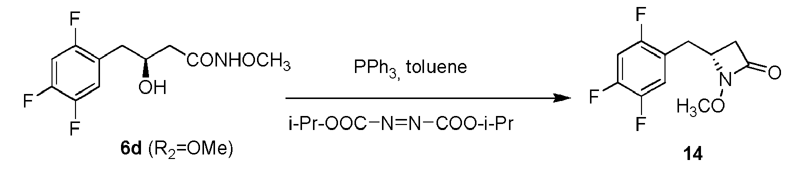

27 g of triphenylphosphine were added in portions to the solution of 19.2 mL diisopropyl azodicarboxylate (DIAD) and 320 mL of toluene, applying external cooling to maintain temperature of the reaction mixture at 19 °C. Then 20.0 g of compound of formula **6d** were divided in three equal portions were added in 10 minutes intervals, maintaining the temperature at 19 °C. After the addition, the reaction mixture was stirred for 3 hours at 19 °C and for 16 hours at 0 °C. The precipitate was filtered-off and discarded. The filtrate was subjected to evaporation at 50 °C and 5 mbars, obtaining 40.3 g of crude product in the form of reddish-brown heavy syrup. It contained side products, which were efficiently purified in the next step.

For analytical purposes, small amount of crude product was purified on silica, applying EtOAc/n-hexane mobile phase. R_{f} = 0.18 (EtOAc/n-hexane = 1/2 (vol.))

¹H NMR (DMSO-d₆): δ 2.43(dd,1H), 2.76(dd,1H), 2.84(dd,1H), 3.13(dd,1H), 3.65(s,3H), 4.06-4.09(m,1H), 7.50-7.60(m,2H)

### Example 21: Preparation of (R)-3-(methoxyamino)-4-(2,4,5-trifluorophenyl)butanoic acid (formula 15, R₂=OMe)

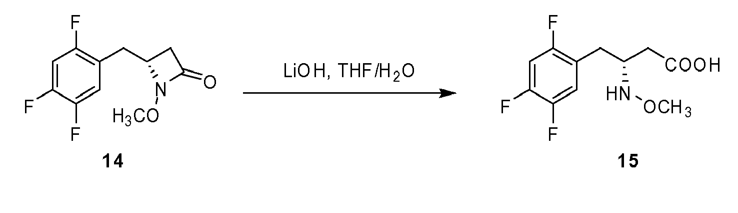

40.2 g of the crude product from previous experiment were suspended in the mixture of 193 mL THF, 97 mL water and 6.9 g lithium hydroxide hydrate. The reaction mixture was stirred for 1.5 hour at 35 °C, followed by evaporation at 35 °C to 40 °C and 35 mbar of final pressure, obtaining ca 138.6 g of residue. To the residue 200 mL of water were added and pH of the reaction mixture was adjusted to 11 with 6 N HCl (cca 10 mL). The reaction mixture was washed twice with 80 mL i-propyl acetate. The water phase was acidified to pH 4 with 6 N HCl and extracted with 2 x 80 mL i-propyl acetate (IPAC). Combined IPAC extracts were washed with the mixture of 45 mL of water and 5 mL of brine, followed by evaporation at 53 °C and 5 mbar, providing 16.4 g of clear brownish syrupy product.

¹H NMR (DMSO-d₆): δ 2.20 (dd,1H), 2.36 (dd,1H), 2.62 (dd,1H), 2.80 (dd, 1H), 3.36 (s, 3H), 3.36-3.44 (m, 1H), 6.45 (s, wide, 1H) 7.36-7.49 (m, 2H), 12.11 (s,wide,1H)

### Example 22: Preparation of ((R)-3-(methoxyamino)-1-(3-(trifluoromethyl)-5,6-dihydro-[1,2,4]triazolo[4,3-a]pyrazin-7(8H)-yl)-4-(2,4,5-trifluorophenyl)butan-1-one (formula 16, R₂=OMe)

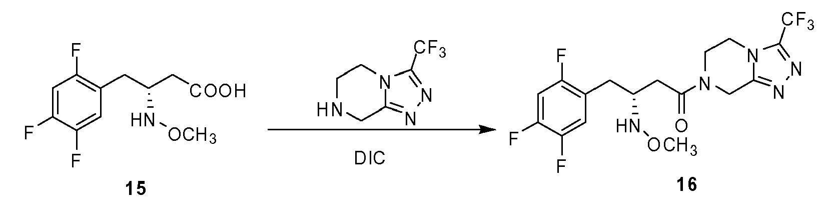

6.0 g of compound of formula **15**, 0.3 g 1-hydroxybenzotrizole hydrate (HOBt) and 4.7 g 3-(trifluoro-methyl)-5,6,7,8-tetrahydro-[1,2,4]triazolo[4,3-a]pyrazine were dissolved in 40 mL of dry dichloromethane. To the clear solution 4.5 mL of N,N'-diisopropylcarbodiimide were added during stirring at 20 °C. After addition, the reaction mixture was kept stirring for 2 hours at 23 °C, followed by cooling the reaction mixture to 0 °C for 2 hours. The precipitate was filtered-off and discarded. The filtrate was evaporated at 45 °C and 5 mbar of final pressure, obtaining 12.0 g of crude product.

### Example 23: Preparation of (R)-3-amino-1-(3-(trifluoromethyl)-5,6-dihydro-[1,2,4]triazolo[4,3-a]pyrazin-7(8H)-yl)-4-(2,4,5-trifluorophenyl)butan-1-one (compound of formula 1; sitagliptin)

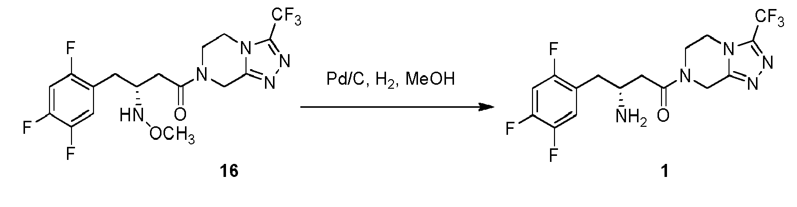

1.35 g of the crude compound of formula **16** from previous experiment and 0.40 g of 5% palladium on carbon are suspended in 20 mL methanol. The suspension is subjected to hydrogenation at 65 °C and 10 bars of pressure for 4 hours. The catalyst is filtered-off, followed by evaporation of filtrate at 55 °C and 10 mbar. 1.23 g of the crude product of formula 1 is obtained. It is dissolved in the mixture of 12 mL isopropanol, 3 mL methanol and 0.20 mL water. During stirring, 0.32 g 85% aqueous phosphoric acid is added. When crystallization starts, the suspension is aged for 20 minutes at -20 °C and filtered. The precipitate is washed on filter with 2 mL of ethanol and 5 mL of diethyl ether, yielding 0.31 g of the product.

¹H NMR (DMSO-d₆, 80°C): δ 2.68-2.93(m,4H), 3.60-3.66(m,1H), 3.94-4.11(m,2H), 4.11-4.32(m,2H), 4.83-5.04(m,2H), 6.76(s/wide/,4H), 7.33-7.39(m,1H), 7.51-7.58(m,1H)

## Claims

1. A process of preparing a compound of formula IV, 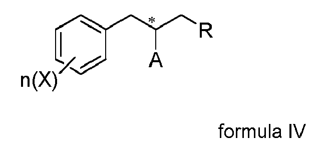 wherein X is halogen selected from fluoro, chloro, or bromo, preferably fluoro, same or different, and n is 1-4, A is OH or NH₂, * denotes a chiral atom and R is selected from
a leaving group or a group convertible to a leaving group;
the process comprising the steps of:
(i) providing a compound of formula II, 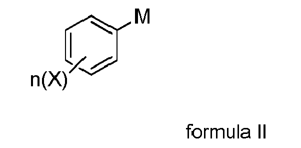 wherein X and n are as defined above and M is a metal element or an organometal group;
(ii) reacting of the compound of formula II with a compound of formula IIIa when A is OH or with a compound of formula IIIb when A is NHP, wherein P is an amine protecting group, 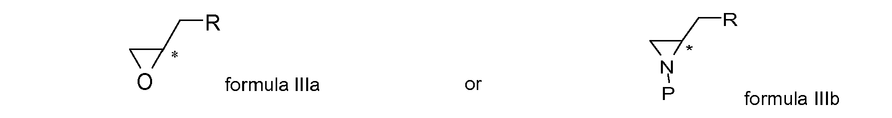 wherein * denotes a chiral atom and R is as defined above.

2. The process according to claim 1, wherein the compound of formula II is defined by formula IIa, 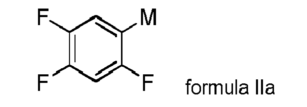 wherein M is as defined above, and wherein the compound of formula IIa is provided by treating a halo-trifluorobenzene, wherein "halo" means chloro, bromo or iodo, preferably chloro, with a metal or an organometal compound of M to form a compound of formula II with X being F and n being 3.

3. The process according to claim 2, wherein the reaction to obtain the compound of formula IIa and the reaction of the compound of formula IIa with the compound of formula IIIa proceed in one pot.

4. The process according to any of the preceding claims, wherein R is
a leaving group selected from the group consisting of halogen, arylsulfonyloxy, unsubstituted and fluorinated C₁-C₄-alkylsulfonyloxy; or
a group convertible to a leaving group, selected from protected hydroxy groups, and inert organometal groups convertible to cyano, aminocarbonyl, hydroxycarbonyl or alkoxycarbonyl groups.

5. The process according to any of the preceding claims, wherein M is or contains magnesium, zinc or lithium, preferably M is halomagnesio.

6. The process according to any of the proceeding claims, wherein the compound of formula IIIa is in the (S)-configuration, preferably (S)-epichlorohydrin is used as the compound of formula IIIa to obtain 1-chloro-3-(2,4,5-trifluorophenyl)-2(S)-propanol as the compound of formula IV.

7. The process according to any one of claims 1 to 4, wherein the compound of formula IV with A being OH, 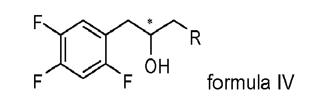 wherein * denotes a chiral atom and R is a leaving group selected from halogen, arylsulfonyloxy, unsubstituted or fluorinated C₁-C₄-alkylsulfonyloxy, preferably chloro,
is further converted to a compound of formula V, 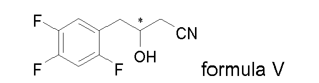 wherein * denotes a chiral atom
by treatment with cyanides, selected from alkali, earth-alkali metalic or quaternary ammonium cyanides.

8. The process according to any one of claims 1 to 4, wherein the compound of formula IV with A being OH, 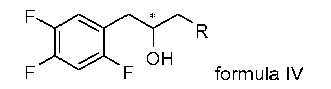 wherein * denotes a chiral atom and R is a hydroxy group, optionally protected to be inert in an organometal containing medium, is further converted to a compound of formula V, 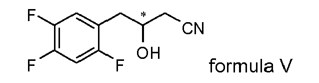 wherein * denotes a chiral atom
said process comprising the steps of:
(i) optionally deprotecting the protected hydroxy group,
(ii) transforming the hydroxyl group to halogen, arylsulfonyloxy, unsubstituted or fluorinated C₁-C₄-alkylsulfonyloxy group, and
(iii) treating the obtained compound of formula IV with cyanides selected from alkali, earth-alkali metalic or quaternary ammonium cyanides, preferably by treatment with potassium or sodium cyanide in a protic solvent or in a mixture of protic and aprotic solvent.

9. The process according to claim 7 or 8, wherein the compound of formula V is further converted to a compound of formula VI, 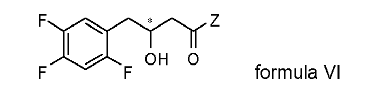 wherein * denotes a chiral atom and Z is selected from the group consisting of hydroxy, amino, OR₁, wherein R₁ is selected from C₁-C₆-alkyl and halo substituted C₁-C₆-alkyl, by treatment of the compound of formula V with strong acid.

10. The process according to any one of claims 7 or 8, wherein the compound of formula V or a salt thereof is further converted to a compound of formula VIc, 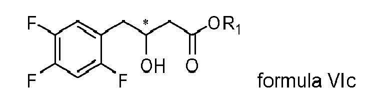 wherein * denotes a chiral atom and R₁ is selected from the group consisting of C₁-C₆-alkyl or halo substituted C₁-C₆-alkyl,
by treatment with a strong acid in a solvent of formula R₁OH, wherein R₁ is H, C₁-C₆-alkyl or halo substituted C₁-C₆-alkyl, preferably ethanol or methanol, optionally diluted with water or aprotic organic solvent; or
wherein the compound of formula V is further converted to a compound of formula VIa, 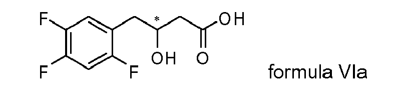 by a treatment selected from (i) to (iii):
(i) treating the compound of formula V in aqueous alkaline conditions, optionally diluted with water miscible solvent, preferably in the presence of hydrogen peroxide, more preferably in concentrated ammonia in the presence of hydrogen peroxide,
(ii) treating the compound of formula V with a strong acid,
(iii) treating the compound of formula VIc, 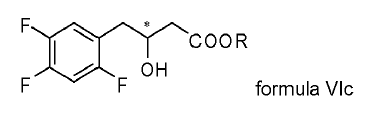
wherein * denotes a chiral and R is C₁-C₆-alkyl or halo substituted C₁-C₆-alkyl, with an aqueous base selected from the group of alkali hydroxides, earth-metal hydroxides and organic bases, preferably sodium hydroxide in C₁-C₄-alkanol/water mixture, most preferably sodium hydroxide in a methanol/water mixture.

11. The process according to claim 10, wherein the compound of formula VIa is further converted to a compound of formula VId, 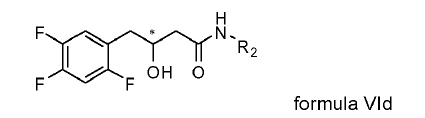 wherein * denotes a chiral atom and R₂ is selected from C₁-C₄-alkyl, aryl-C₁-C₂-alkyl, C₁-C₄-alkoxy, aryl-C₁-C₂-alkoxy, trifluoroacetyl, preferably methoxy,
by treatment with a coupling reagent selected from carbodiimides and amines R₂-NH₂, wherein R₂ is as defined, in the presence of a solvent.

12. The process according to claim 9, further comprising the steps of:
(i) reacting the compound of formula VI, wherein Z is hydroxyl, with a heterocyclic moiety of formula VIII 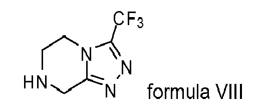 in the presence of coupling reagent in an organic solvent, forming a compound of formula VII or a salt thereof, 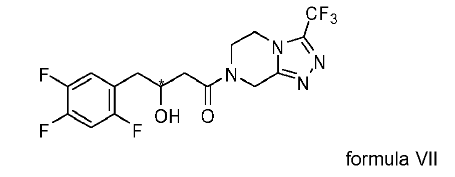 wherein * denotes a chiral atom
(ii) sulfonation of the compound of formula VII or a salt thereof with a halogenide or an anhydride of a sulfonic acid in the presence of a base in an aprotic organic solvent,
to obtain a compound of formula IX or a salt thereof, 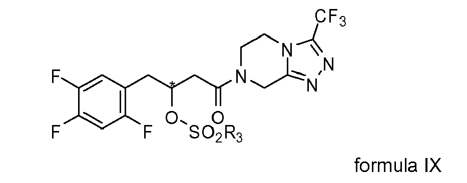 wherein * denotes a chiral atom and R₃ is selected from methyl, ethyl, benzyl, p-methylbenzyl, p-bromobenzyl, p-nitrobenzyl and 10-camphoryl,
optionally wherein the compound of formula IX is further converted to a compound of formula X, 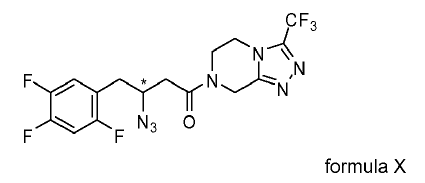 wherein * denotes a chiral atom.

13. The process according to claim 9, further comprising sulfonation of the compound of formula VI with a halogenide or an anhydride of a sulfonic acid in the presence of a base in an aprotic organic solvent, to obtain a compound of formula XI, 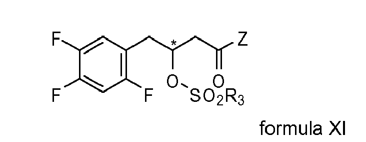 wherein * denotes a chiral atom and Z is selected from the group consisting of OR₁, wherein R₁ is selected from C₁-C₆-alkyl and halo substituted C₁-C₆-alkyl, and NHR₂, wherein R₂ is selected from H, C₁-C₄-alkyl, aryl-C₁-C₂-alkyl, C₁-C₄-alkoxy, aryl-C₁-C₂-alkoxyand trifluoroacetyl, and R₃ is selected from methyl, ethyl, benzyl, p-methylbenzyl, p-bromobenzyl, p-nitrobenzyl and 10-camphoryl,
optionally wherein the compound of formula XI is further converted to a compound of formula XII, 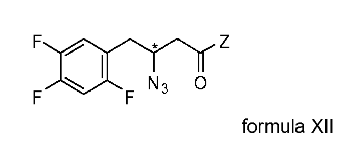 wherein * denotes a chiral atom and R₂ is selected from C₁-C₆-alkyl, aryl-C₁-C₄-alkyl, C₁-C₄-alkoxyand trifluoroacetyl,
by treatment with an azide reagent, selected from alkali metal azide or trialkylammonium azide in a solvent, selected from aprotic polar solvents;
or optionally wherein the compound of formula XI is further converted to a compound of formula XIV, 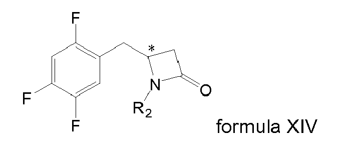 wherein * denotes a chiral atom and R₂ is selected from C₁-C₆-alkyl, C₁-C₄-alkoxy and trifluoroacetyl,
by cyclization of in the presence of strong base, preferably sodium hydride, in a solvent selected from amides, halogenated alkanes and aromatic hydrocarbons.

14. A compound of formula IV, 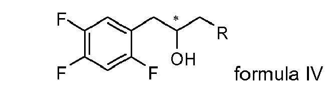 wherein * denotes a chiral atom and R is selected from leaving groups or groups convertible to a leaving group.

15. A compound selected from the group consisting of:
a compound of formula VI, 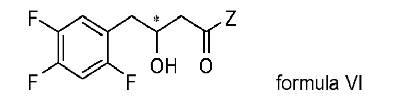
wherein * denotes a chiral atom and Z is selected from the group consisting of OR₁, wherein R₁ is selected from C₁-C₆-alkyl and halo substituted C₁-C₆-alkyl, NHR₂, wherein R₂ is selected from H, C₁-C₄-alkyl, aryl-C₁-C₂-alkyl, C₁-C₄-alkoxy, aryl-C₁-C₂-alkoxy and trifluoroacetyl;
a compound of formula XI, 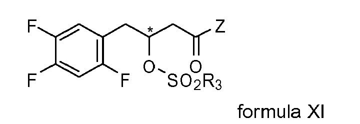
wherein * denotes a chiral atom and Z is selected from the group consisting of OR₁, wherein R₁ is selected from C₁-C₆-alkyl or halo substituted C₁-C₆-alkyl,
NHR₂, wherein R₂ is selected from H, C₁-C₄-alkyl, aryl-C₁-C₂-alkyl, C₁-C₄-alkoxy, aryl-C₁-C₂-alkoxy, trifluoroacetyl and R₃ is selected from methyl, ethyl, benzyl, p-methylbenzyl, p-bromobenzyl, p-nitrobenzyl and 10-camphoryl;
a compound of formula XIV, 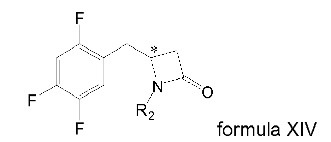
wherein * denotes a chiral atom and R₂ is selected from C₁-C₆-alkyl, C₁-C₄-alkoxyl and trifluoroacetyl;
a compound of formula XVII, 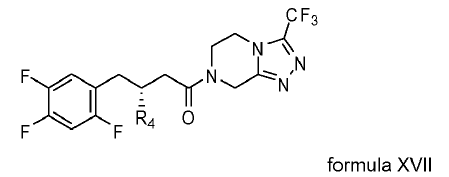
wherein R₄ is selected from azido and NHOMe.

16. A process for producing sitagliptin or a salt thereof, comprising the steps of carrying out a process for preparing the compound of formula IV according to claim 1, or carrying out a process for preparing the compound of formula VI according to claim 9,
and subjecting the compound of formula IV or the compound of formula VI to further synthesis steps to yield sitagliptin or a salt thereof.

17. Use of a compound of formula IV, 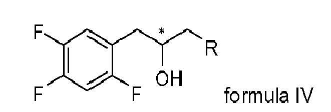 wherein * denotes a chiral atom and R is selected from leaving groups or groups convertible to a leaving group, for the synthesis of gliptins or salts thereof.

18. Use of a compound according to claim 15 for the synthesis of gliptins or salts thereof.

19. A process for producing a pharmaceutical composition comprising sitagliptin as active ingredient, comprising the steps of:
(i) preparing sitagliptin or a salt thereof according to a process as defined in claim 16, and
(ii) admixing the thus prepared sitagliptin with at least one pharmaceutically acceptable excipient.
